# EUROPEAN PATENT APPLICATION

(11) **EP 2 939 671 A1**
(43) Date of publication of application: **04.11.2015**
(21) Application number: 15162676.9
(22) Date of filing: 02.02.2010
(51) Int. Cl.: A61K 31/202, A61P 9/00

(54) **METHODS FOR IMPROVING COGNITIVE FUNCTION AND DECREASING HEART RATE**

(30) Priority: 02.02.2009 US 149310 P; 02.06.2009 US 183548 P
(62) Divisional of application: 10703592.5
(71) Applicant: DSM IP Assets B.V., 6411 TE Heerlen (NL)
(72) Inventor: Yurko-Mauro, Karin, Columbia, MD 21045 (US)
(74) Representative: D Young & Co LLP

(57) **Abstract**

The present invention is directed to methods of improving cognitive function in subjects having age related cognitive decline or mild cognitive impairment and to methods of decreasing heart rate in a subject by administering dosage forms comprising docosahexaenoic acid (DHA) substantially free of eicosapentaenoic acid (EPA).

## Description

### CROSS-REFERENCE TO RELATED APPLICATIONS

This application claims benefit under 35 U.S.C. § 119(e) of United States application Serial No. 61/149,310, filed February 2, 2009, and United States application Serial No. 61/183,548, filed June 2, 2009, the contents of which are incorporated herein by reference.

### TECHNICAL FIELD

The present disclosure is directed to methods of improving cognitive function in subjects having age related cognitive decline or mild cognitive impairment and/or reducing heart rate by administering dosage forms comprising docosahexaenoic acid (DHA) substantially free of eicosapentaenoic acid (EPA).

### BACKGROUND

Age-related cognitive impairment is considered a strong risk factor for the development of dementia. As the populations of elderly people grow, and effective curative approaches are not yet available, it is of major importance to develop and study preventative and ameliorative measures.

Previous studies have speculated that increased intake of fish and n-3 polyunsaturated fatty acids (PUFAs) may play a protective role against age-related cognitive decline. Additional studies have shown that decreases in plasma docosahexaenoic acid (DHA), the principle long chain omega-3 fatty acid in brain, are associated with cognitive decline in healthy elderly subjects, and in cognitively impaired subjects, with and without Alzheimer's disease. See e.g., Nelson et al., Lipids 32:11291136 (1997); Knapp et al., N Engl. J Med. 320:1037-1043 (1989); and Bonaa et al., N. Engl. J Med. 322:795-801 (1990). DHA plays an important role in neural and visual development and multiple brain functions. Decreases in plasma DHA are associated with cognitive decline in healthy elderly and Alzheimer's patients. See e.g., Heude et al., Am. J Clin. Nutr. 77:803-808 (2003); Tully et al., Br. J Nutr. 89:483-490 (2003). Greater DHA intake and greater plasma DHA levels are inversely correlated with relative risk of incident Alzheimer's disease (AD) and all-cause dementia. See e.g., Morris et al., Arch. Neurol. 60:194-200 (2003); Schaefer, et al., Arch. Neurol. 63:1545-1550 (2006). Administration of algal sources of DHA triglyceride oil reduced insoluble amyloid (70%), Aβ42 levels (49%), and plaque burden (40%) in the aged APPsw (Tg2576) mouse model of Alzheimer's disease and reduced Aβ and tau levels in the 3xTg-AD mouse. See e.g., Calon et al., Neuron 43:633-645 (2004); Lim et al., J. Neurosci. 25:3032-40 (2005); and Green et al., J. Neurosci. 27:4385-95 (2007).

### SUMMARY

In one aspect, the present disclosure is directed to a method of improving cognitive function in a subject with mild cognitive impairment or age related cognitive decline, comprising administering to the subject in need thereof about 0.8 g to about 4 g of docosahexaenoic acid (DHA) per day wherein the DHA is provided substantially free of eicosapentaenoic acid (EPA)

In another aspect, the present disclosure is directed to a method of decreasing heart rate in a subject, comprising administering to the subject in need thereof about 0.8 g to about 4 g of docosahexaenoic acid (DHA) per day wherein the DHA is provided substantially free of eicosapentaenoic acid (EPA). In some embodiments, the subject for treatment to reduce the heart rate may or may not have associated mild cognitive impairment or age related cognitive decline.

In some embodiments, the DHA is provided substantially free of arachidonic acid (ARA).

In some embodiments, the dosage form is administered to the subject in at least one unit dose. In some embodiments, the unit dose comprises about 0.2 g to about 1 g of DHA.

### BRIEF SUMMARY OF THE FIGURES

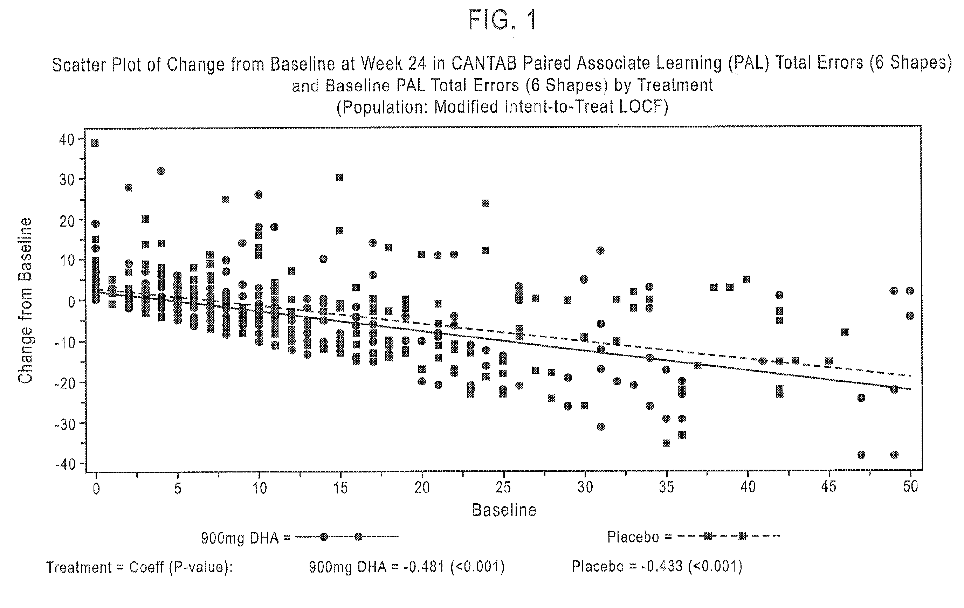

FIG. 1 represents a scatter plot of change from baseline at Week 24 in CANTAB Paired Associate Learning (PAL) Total Errors (6 Shapes) and Baseline PAL Total Errors (6 Shapes) by Treatment.

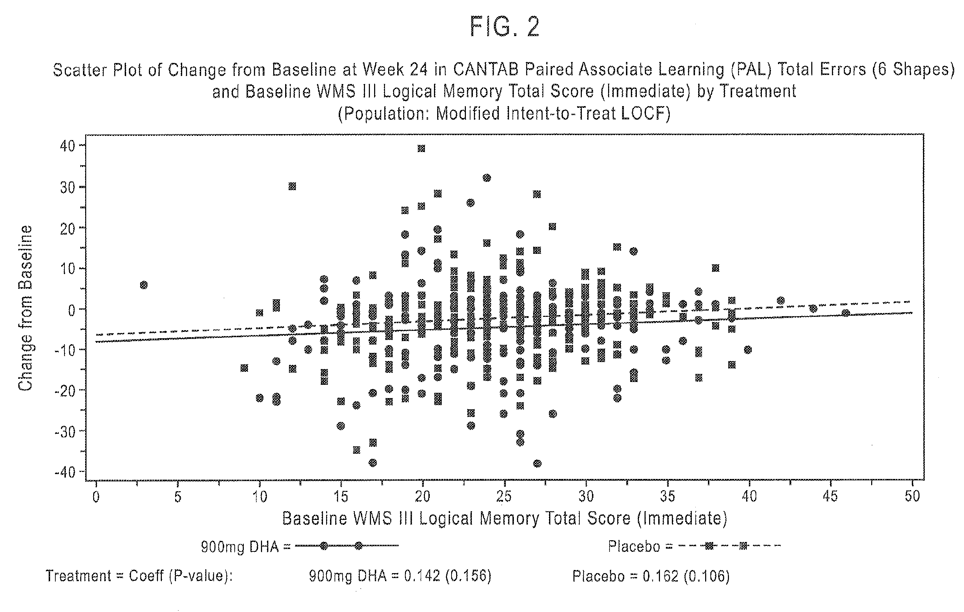

FIG. 2 represents a scatter plot of change from baseline at Week 24 in CANTAB Paired Associate Learning (PAL) Total Errors (6 shapes) and Baseline WMS III Logical Memory Total Score (Immediate) by Treatment (Population: Modified Intent-to-Treat LOCF).

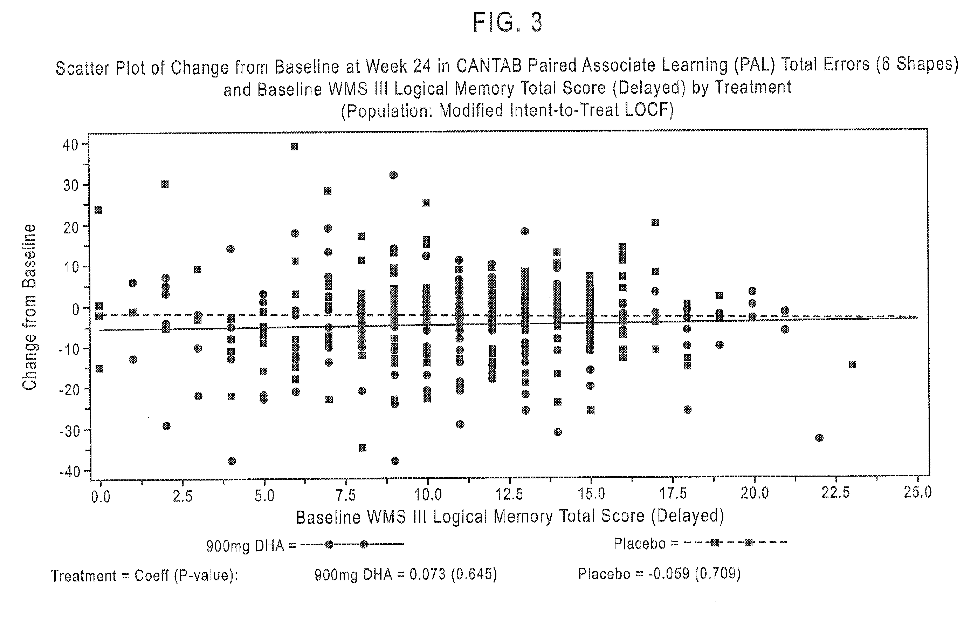

FIG. 3 represents a scatter plot of change from baseline at Week 24 in CANTAB Paired Associate Learning (PAL) Total Errors (6 shapes) and Baseline WMS III Logical Memory Total Score (Delayed) by Treatment (Population: Modified Intent-to-Treat LOCF).

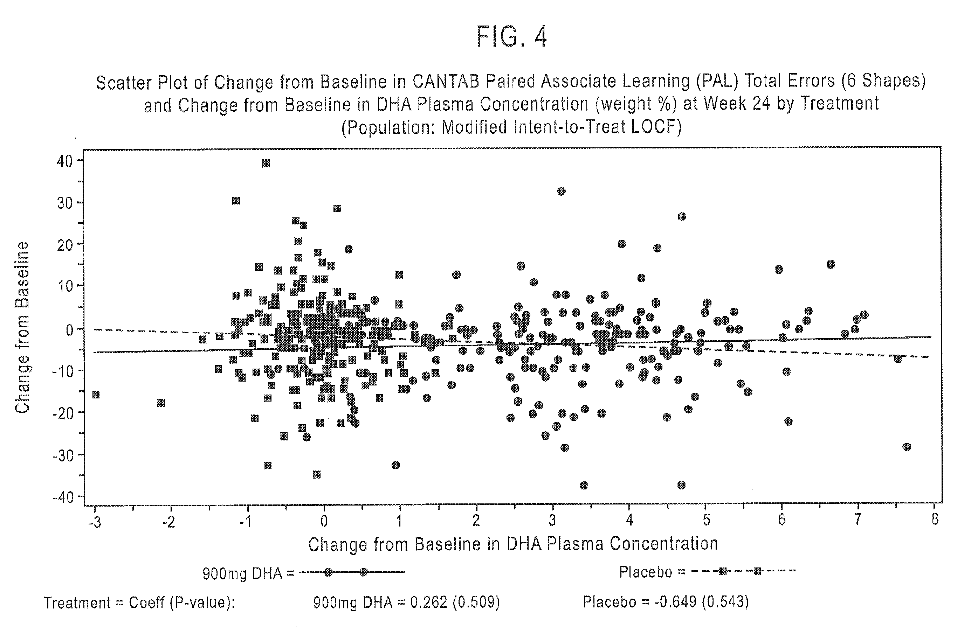

FIG. 4 represents a scatter plot of change from baseline in CANTAB Paired Associate Learning (PAL) Total Errors (6 shapes) and log Change from Baseline in DHA Plasma Concentration (weight %) at Week 24 by Treatment (Population: Modified Intent-to-Treat LOCF).
FIG. 5A is a Table representing the Primary Efficacy Analysis of the DHA regimen: Summary of Change from Baseline in CANTAB Paired Associate Learning (PAL) Total Errors (6 Shapes) at Week 24 (Population: Modified Intent-to-Treat LOCF). FIG. 5B represents the Secondary Efficacy Analysis of the DHA regimen: Summary of Change from Baseline in other Cognitive and Functional tests at Week 24 (Population: Modified Intent-to-Treat LOCF).
FIG. 6 is a Table representing the Primary Efficacy Analysis of the DHA regimen: Summary of Change from Baseline in CANTAB Paired Associate Learning (PAL) Total Errors (6 Shapes) at Week 24 (Population: Per Protocol LOCF).
FIG. 7 is a Table representing the Primary Efficacy Analysis: Summary of Change from Baseline in CANTAB Paired Associate Learning (PAL) Total Errors (6 Shapes) at Week 24 (Population: Intent-to-Treat LOCF).

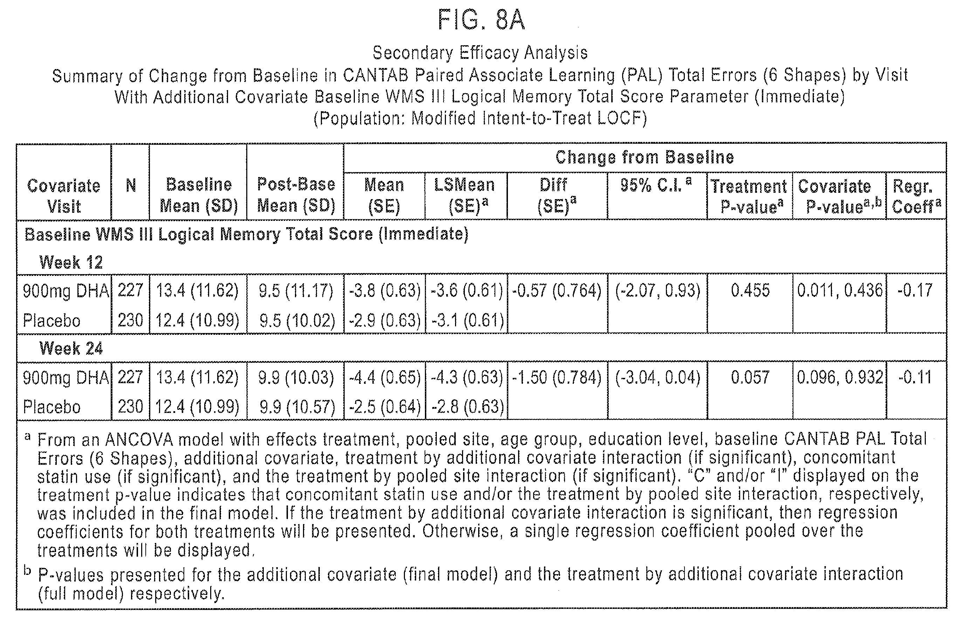

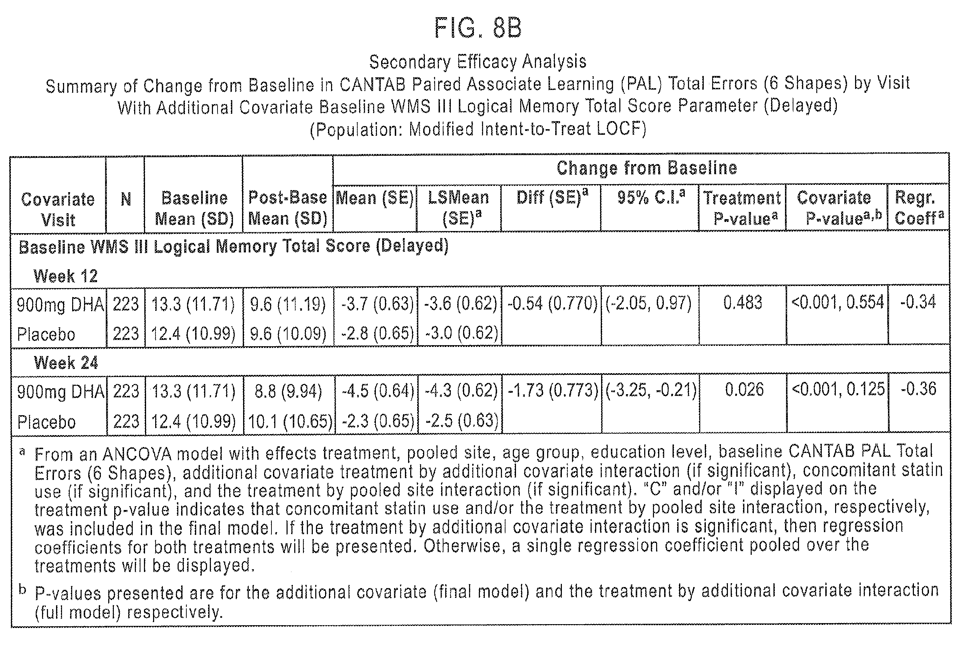

FIGS. 8A and 8B represent the Secondary Efficacy Analysis: Summary of Change from Baseline in CANTAB Paired Associate Learning (PAL) Total Errors (6 Shapes) by Visit With Additional Covariate Baseline WMS III Logical Memory Total Score Parameter (Immediate or Delayed) (Population: Modified Intent-to-Treat LOCF). FIG. 8A = immediate memory, and FIG. 8B = delayed memory.

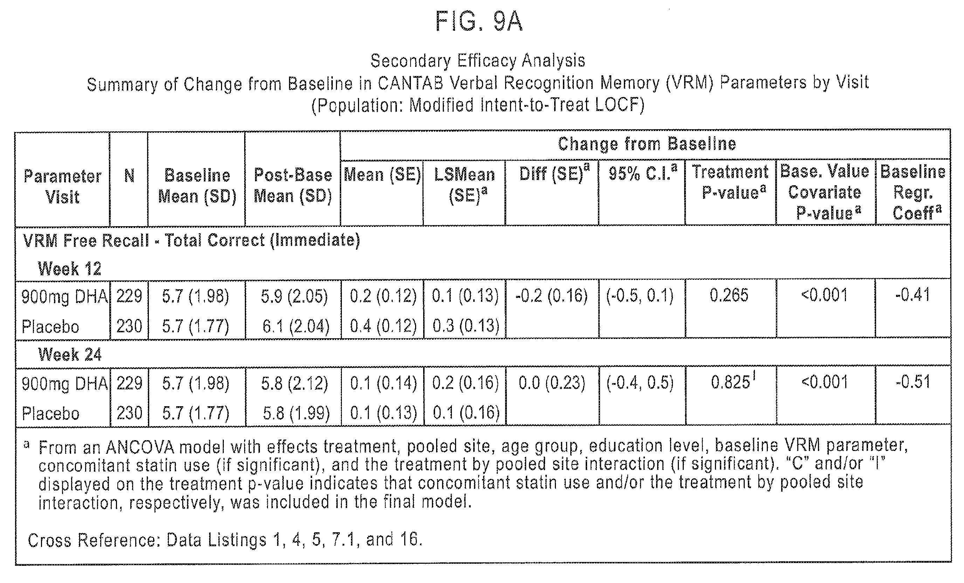

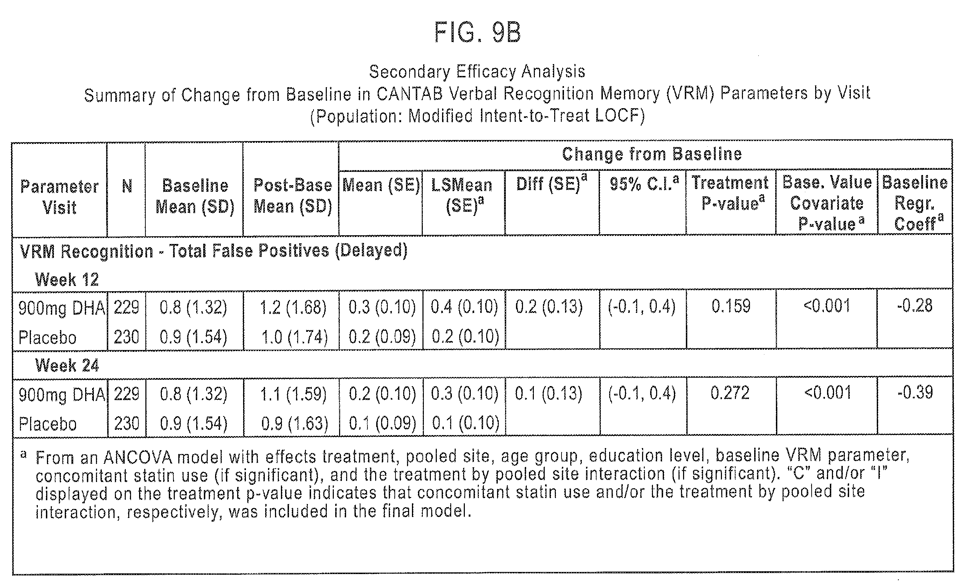

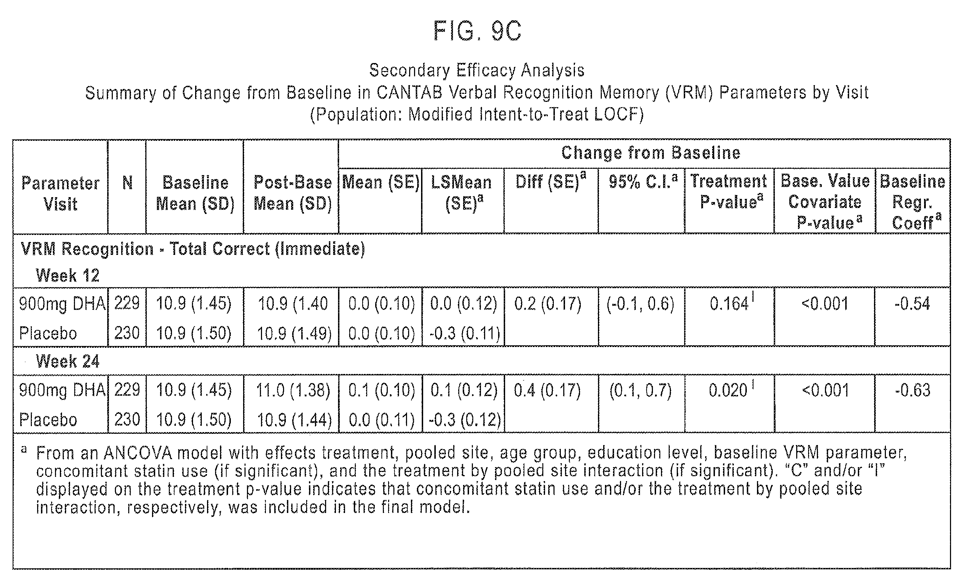

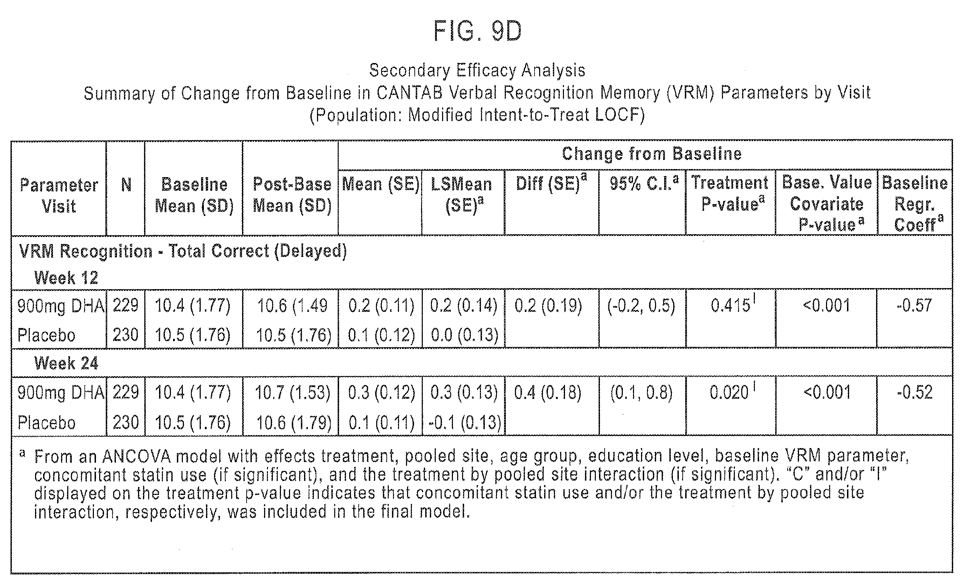

FIGS. 9A to 9D represent the Secondary Efficacy Analysis: Summary of Change from Baseline in CANTAB Verbal Recognition Memory (VRM) Parameters by Visit (Population: Modified Intent-to-Treat LOCF). FIG. 9A = VRM Free Recall - Total Correct (Immediate); FIG. 9B = VRM Total False Positives (Delayed); FIG. 9C = VRM Recognition - Total Correct (Immediate); and FIG. 9D = VRM Recognition - Total Correct (Delayed).
FIG. 10 is a Table representing the plasma phospholipid fatty acid levels III subjects administered about 900 mg/d DHA or a placebo for 24 weeks.
FIG. 11 is a Table which shows the change from baseline in select efficacy and safety parameters with log week 24 DHA plasma concentration (weight %), including a change from baseline in heart rate.

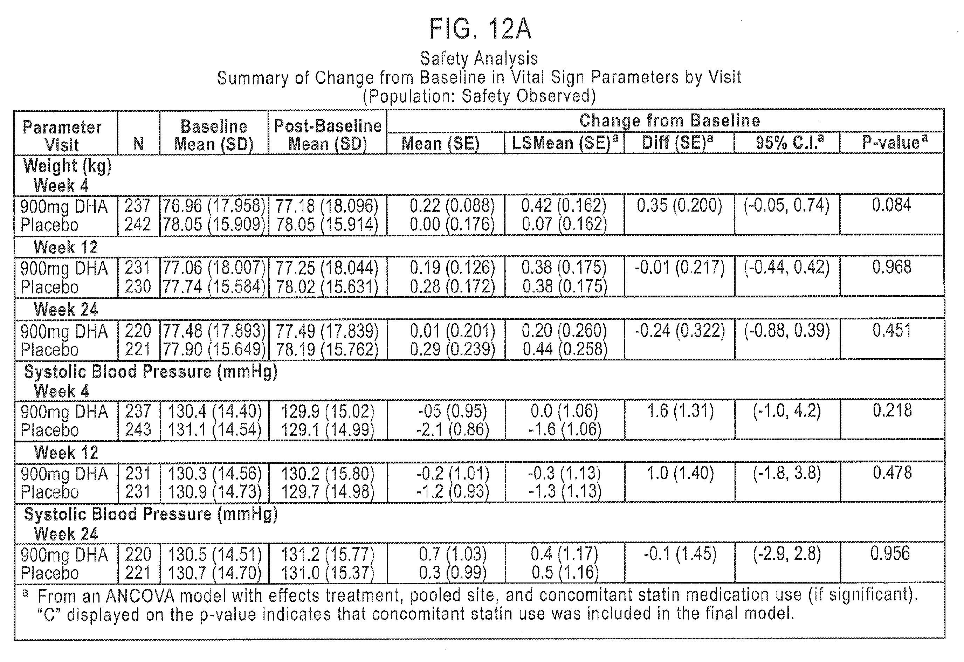

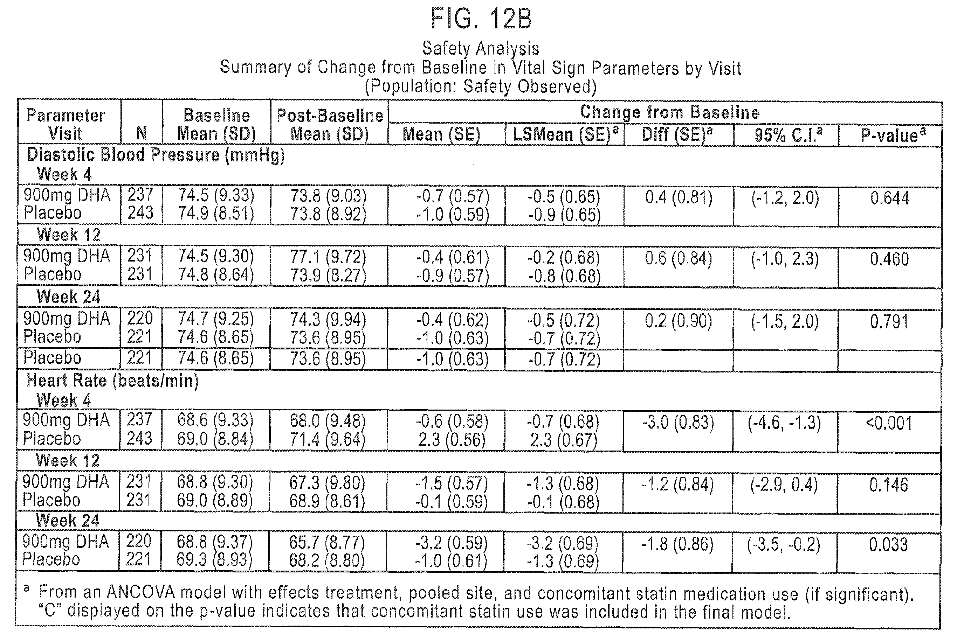

FIGS. 12A and 12B are tables which show the safety analysis: Summary of change from a baseline in vital sign parameters by visit (Population safety observed). FIG. 12A = weight and systolic blood pressure, and FIG. 12B = diastolic blood pressure and heart rate.

### DETAILED DESCRIPTION

The present disclosure provides methods of improving memory, cognitive function and/or decreasing heart rate in a subject, comprising administering to the subject in need thereof about 0.8 g up to about 4 g of DHA, where the DHA is provided substantially free of EPA. As further discussed below, in some embodiments, the methods provided herein relate to treatment of a subject with mild cognitive impairment or age related cognitive decline to improve cognitive function or reduce the severity of cognitive impairment or cognitive decline, the method comprising administering to the subject in need thereof about 0.8 g up to about 4 g of DHA, where the DHA is provided substantially free of EPA.

Administration of DHA is also shown herein to reduce the heart rate of treated subjects. Accordingly, in some embodiments, the administration of DHA can also be used to reduce the heart rate of a subject, particularly those that would benefit from reduced cardiac load, the method comprising administering to the subject in need thereof about 0.8 g up to about 4 g of DHA, where the DHA is provided substantially free of EPA. In some embodiments, DHA can be used to treat a subject afflicted with mild cognitive impairment or age related cognitive decline in a subject and concurrently used to reduce the heart rate of the subject, the method comprising administering to the subject in need thereof about 0.8 g up to about 4 g of DHA, where the DHA is provided substantially free of EPA.

Accordingly, in some embodiments, the present disclosure provides a method of improving cognitive function and/or decreasing heart rate in a subject having mild cognitive impairment or age related cognitive decline, comprising administering to a subject in need thereof about 0.8 g up to about 4 g of DHA, where the DHA is provided substantially free of EPA. In some embodiments, the dosage form is also substantially free of arachidonic acid (ARA).

As further discussed below, in some embodiments, the dose of DHA administered to the subject is about 0.8 g to about 4 g per day. In some embodiments, the dose administered is about 0.84 g to about 4 g of DHA per day. In some embodiments, the dose administered is about 0.84 g to about 1.5 g of DHA per day. In some embodiments, the dose administered is about 0.84 mg to about 1.0 g of DHA per day. In some embodiments, the dosage form is a unit dose such that the DHA is administered in at least one unit dose.

In the methods herein, "docosahexaenoic acid" and "DHA" are used interchangeably herein to refer to the compound with the chemical name (all-Z)-4,7,10,13,16,19-docosahexaenoic acid, as well as any salts or derivatives thereof. Thus, DHA encompasses the free acid DHA as well as DHA esters and triglycerides containing DHA. DHA is an ω-3 polyunsaturated fatty acid. Hence, in various embodiments, the DHA used in the method may be in the form of a phospholipid, a triglyceride, free fatty acid, and/or an ester.

In some embodiments, the DHA can be in a mono, di or triglyceride form. For example, one, two or three DHA molecules can be in the mono, di or triglyceride molecule.

In some embodiments, the DHA can be in the form of an ester. The term "ester" refers to the replacement of the hydrogen in the carboxylic acid group of the DHA molecule with another substituent. Typical esters are known to those in the art, a discussion of which is provided by Higuchi, T. and V. Stella in Pro-drugs as Novel Delivery Systems, Vol. 14, A.C.S. Symposium Series, Bioreversible Carriers in Drug Design, Ed. Edward B. Roche, American Pharmaceutical Association, Pergamon Press, 1987, and Protective Groups in Organic Chemistry, McOmie ed., Plenum Press, New York, 1973. Examples of the most common esters include methyl, ethyl, propyl, butyl, pentyl, t-butyl, benzyl, nitrobenzyl, methoxybenzyl, benzhydryl, and trichloroethyl. In some embodiments, the ester is a carboxylic acid protective ester group, esters with aralkyl (e.g., benzyl, phenethyl), esters with lower alkenyl (e.g., allyl, 2-butenyl), esters with lower-alkoxy-lower-alkyl (e.g., methoxymethyl, 2-methoxyethyl, 2-ethoxyethyl), esters with lower-alkanoyloxy-lower-alkyl (e.g., acetoxymethyl, pivaloyloxymethyl, 1-pivaloyloxyethyl), esters with lower-alkoxycarbonyl-lower-alkyl (e.g., methoxycarbonylmethyl, isopropoxycarbonylmethyl), esters with carboxy-lower alkyl (e.g., carboxymethyl), esters with lower-alkoxycarbonyloxy-lower-alkyl (e.g., 1-ethoxycarbonyloxy)ethyl, 1-(cyclohexyloxycarbonyloxy)ethyl), esters with carbamoyloxy-lower alkyl (e.g., carbamoyloxymethyl), and the like. In some embodiments, the added substituent is a linear or cyclic hydrocarbon group, e.g., a C₁-C₆ alkyl, C₁-C₆ cycloalkyl, C₁-C₆ alkenyl, or C₁-C₆ aryl ester.

In some embodiments, the DHA ester is an alkyl ester, e.g., a methyl ester, ethyl ester or propyl ester. More particularly, the ester is an ethyl ester. In some embodiments, the ester substituent is added to the DHA free acid molecule when the DHA is in a purified or semi-purified state. Alternatively, the DHA ester is formed upon conversion of a triglyceride to an ester. One of skill in the art can appreciate that some non-esterified DHA molecules may be present in the present invention, e.g., DHA molecules that have not been esterified, or DHA linkages that have been cleaved, e.g., hydrolyzed. In some embodiments, the non-esterified DHA molecules constitute less than 3% (mol/mol), about 2% to about 0.01% (mol/mol), about 1% to about 0.05% (mol/mol), or about 5% to about 0.1% (mol/mol) of the total DHA molecules. Alternatively, in some embodiments, the DHA can be in a free acid form and/or in a salt form.

The DHA can be derived from various sources, e.g., from oleaginous microorganisms. As used herein, "oleaginous microorganisms" are defined as microorganisms capable of accumulating greater than 20% of the dry weight of their cells in the form of lipids. In some embodiments, the DHA is derived from a phototrophic or heterotrophic single cell organism or multicellular organism, e.g., an algae. For example, the DHA can be derived from a marine algae, such as Crypthecodinium sp., Thraustochytrium sp., Schizochytrium sp., or combinations thereof. Thus, in some embodiments the DHA is from an algal source. In some embodiments, the algal source is Crypthecodinium cohnii or Schizochytrium sp.

The source of the DHA can include a microbial source, including the microbial groups Stramenopiles, Thraustochytrids, and Labrinthulids. Stramenopiles includes microalgae and algae-like microorganisms, including the following groups of microorganisms: Hamatores, Proteromonads, Opalines, Develpayella, Diplophrys, Labrinthulids, Thraustochytrids, Biosecids, Oomycetes, Hypochytridiomycetes, Commation, Reticulosphaera, Pelagomonas, Pelagococcus, Ollicola, Aureococcus, Parmales, Diatoms, Xanthophytes, Phaeophytes (brown algae), Eustigmatophytes, Raphidophytes, Synurids, Axodines (including Rhizochromulinaales, Pedinellales, Dictyochales), Chrysomeridales, Sarcinochrysidales, Hydrurales, Hibberdiales, and Chromulinales. The Thraustochytrids include the genera *Schizochytrium* (species include *aggregatum, limnaceum, mangrovei*, *minutum, octosporum*)*, Thraustochytrium* (species include *arudimentale*, *aureum, benthicola, globosum, kinnei*, *motivum, multirudimentale, pachydermum*, *proliferum, roseum, striatum*)*, Ulkenia* (species include *amoeboidea, kerguelensis, minuta, profunda, radiate, sailens, sarkariana*, *schizochytrops*, *visurgensis*, *yorkensis*), *Aplanochytrium* (species include *haliotidis, kerguelensis*, *profunda, stocchinoi*), *Japonochytrium* (species include *marinum*), *Althornia* (species include *crouchii),* and *Elina* (species include *marisalba, sinorifica*). The Labrinthulids include the genera *Labyrinthula* (species include *algeriensis*, *coenocystis, chattonii, macrocystis, macrocystis αtlantica, macrocystis macrocystis*, *marina, minuta, roscofJensis, valkanovii, vitellina, vitellina pacifica, vitellina vitellina, zopfi*)*, Labyrinthomyxa* (species include *marina*), *Labyrinthuloides* (species include *haliotidis, yonkensis), Diplophrys* (species include *archeri*), *Pyrrhosorus** (species include *marinus*), *Sorodiplophrys** (species include *stercorea*), and *Chlamydomyxa** (species include *labyrinthuloides, montana*) (* = there is no current general consensus on the exact taxonomic placement of these genera).

In some embodiments, the algal source is *Crypthecodinium cohnii.* Samples of *C*. *cohnii*, have been deposited with the American Type Culture Collection at Rockville, MD, and assigned AccessionNos. 40750, 30021, 30334-30348, 30541-30543, 30555-30557, 30571, 30572, 30772-30775, 30812, 40750, 50050-50060, and 50297-50300.

As used herein, the term "microorganism," or any specific type of organism, includes wild strains, mutants or recombinant types, and organisms which can produce an enhanced level of oil containing DHA. Also included are microorganisms designed to efficiently use more cost- effective substrates while producing the same amount of DHA as the comparable wild-type strains. Cultivation of dinoflagellates such as *C. cohnii* has been described previously. See, e.g., U.S. Pat. No. 5,492,938 and Henderson, et al., Phytochemistry 27:16791683 (1988). Organisms useful in the production of DHA can also include any manner of transgenic or other genetically modified organisms, e.g., plants, grown either in culture fermentation or in crop plants, e.g., cereals such as maize, barley, wheat, rice, sorghum, pearl millet, com, rye and oats; or beans, soybeans, peppers, lettuce, peas, Brassica species (e.g., cabbage, broccoli, cauliflower, brussel sprouts, rapeseed, and radish), carrot, beets, eggplant, spinach, cucumber, squash, melons, cantaloupe, sunflowers, safflower, canola, flax, peanut, mustard, rapeseed, chickpea, lentil, white clover, olive, palm, borage, evening primrose, linseed, and tobacco.

Another source of oils containing DHA suitable for the compositions and methods of the present invention includes an animal source. Examples of animal sources include aquatic animals (e.g., fish, marine mammals, and crustaceans such as krill and other euphausids) and animal tissues (e.g., brain, liver, eyes, etc.) and animal products such as eggs or milk. Thus, in some embodiments, the method of the present invention comprises administering daily to the subject a dosage form comprising docosahexaenoic acid (DHA) substantially free of eicosapentaenoic acid (EPA), wherein the DHA is derived from a non-algal source, e.g., fish.

DHA can be purified to various levels. DHA purification can be achieved by any means known to those of skill in the art, and can include the extraction of total oil from an organism which produces DHA. In some embodiments, EPA and/or ARA is then removed from the total oil, for example, via chromatographic methods. Alternatively, DHA purification can be achieved by extraction of total oil from an organism which produces DHA, but produces little, if any, amount of EPA and/or ARA.

Microbial oils useful in the methods herein be recovered from microbial sources by any suitable means known to those in the art. For example, the oils can be recovered by extraction with solvents such as chloroform, hexane, methylene chloride, methanol and the like, or by supercritical fluid extraction. Alternatively, the oils can be extracted using extraction techniques, such as are described in U.S. Pat. No. 6,750,048 and International Pub. No. WO01053512, both filed Jan. 19, 2001, and entitled "Solventless extraction process," both of which are incorporated herein by reference in their entirety.

Additional extraction and/or purification techniques are taught in International Pub. No. WO01076715; International Pub. No. WO01076385; US Pub. No. 20070004678; US Pub. No. 20050129739; US Pat. No. 6,399,803; and International Pub. No. WO01051598; all of which are incorporated herein by reference in their entirety. The extracted oils can be evaporated under reduced pressure to produce a sample of concentrated oil material. Processes for the enzyme treatment of biomass for the recovery of lipids are disclosed in International Pub. No. WO2003092628; US Pub. No. 20050170479; EP Pat Pub. 0776356, and U.S. Pat. No. 5,928,696, entitled "Process for extracting native products which are not water-soluble from native substance mixtures by centrifugal force," all of which are incorporated herein by reference in their entirety.

In some embodiments, the DHA can be prepared as esters using a method comprising: a) reacting a composition comprising polyunsaturated fatty acids in the presence of an alcohol and a base to produce an ester of a polyunsaturated fatty acid from the triglycerides; and b) distilling the composition to recover a fraction comprising the ester of the polyunsaturated fatty acid, optionally wherein the method further comprises: c) combining the fraction comprising the ester of the polyunsaturated fatty acid with urea in a medium; d) cooling or concentrating the medium to form a urea-containing precipitate and a liquid fraction; and e) separating the precipitate from the liquid fraction. See, e.g., US Pub. No. 20090023808, incorporated by reference herein in its entirety. In some embodiments, the purification process includes starting with refined, bleached, and deodorized oil (RBD oil), then performing low temperature fractionation using acetone to provide a concentrate. The concentrate can be obtained by base-catalyzed transesterification, distillation, and silica refining to produce the final DHA product

Methods of determining purity levels of fatty acids are known in the art, and can include, e.g., chromatographic methods such as, e.g., HPLC silver ion chromatographic columns (ChromSpher 5 Lipids HPLC Column, Chrompack, Raritan NJ). Alternatively, the purity level can be determined by gas chromatography, with or without converting DHA to the corresponding methyl ester.

For treating the subject with mild cognitive decline, age related cognitive impairment, and/or for reducing the heart rate, various dosage amounts of DHA can be administered to a subject. The term "daily dosage," "daily dosage level," "daily dosage amount" or "dose per day" refers to the total amount of DHA administered per day (about 24 hour period). Thus, for example, administration of DHA to a subject at a daily dosage of 2 g means that the subject receives a total of 2 g of DHA per day, whether the DHA is administered as a single dosage form comprising 2 g DHA, or alternatively, four dosage forms comprising 500 mg DHA each (for a total of 2 g DHA). In some embodiments, the daily amount or dose per day of DHA is about 4 g DHA or less, about 200 mg to about 3.8 g DHA, about 500 mg to about 3.7 g of DHA, about 750 mg to about 3.5 g DHA, or about 1 g to about 2 g DHA. In some embodiments, the daily amount of DHA is about 520 mg to about 4 g, about 540 mg to about 4 g, about 560 mg to about 4 g, or about 580 mg to 4 g. In some embodiments, the daily amount of DHA is less than about 3.8 g DHA, about 900 mg to about 3.6 g DHA, or about 1.8 g to about 2.7 g of DHA. In some embodiments, the daily amount of DHA comprises about 100 mg, 200 mg, 300 mg, 400 mg, 450 mg, 500 mg, 520 mg, 540 mg, 600 mg, 700 mg, 800 mg, 900 mg, 1 g, 1.5 g, 1.8 g, 2.0 g, 2.5 g, 2.7 g, 3.0 g, 3.2 g, 3.3 g, 3.4 g, 3.5 g, 3.6 g, 3.7 g, 3.8 g, 3.9 g, 4.0 g, 4.5 g, 5.0 g, 6.0 g, 6.5 g, 7 g, 8 g, 9 g, or 10 g DHA.

In some embodiments, the daily amount or dose per day of DHA is about 0.8 g to about 4 g of DHA. In some embodiments, the dose per day of DHA is about 0.84 g to about 4 g of DHA In some embodiments, the dose per day of DHA is about 0.84 g to about 1.5 g of DHA. In some embodiments, the dose per day of DHA is about 0.84 mg to about 1.0 g of DHA.

In some embodiments, the DHA can be administered in a single dosage form, i.e., a unit dose, or in two or more dosage forms (i.e., two or more unit doses). As used herein, a "unit dose" refers to an amount of DHA administered to a subject in a single dosage form, e.g., in a gel capsule. The term "unit dose" can also refer to a unit of pharmaceutically suitable liquid, syrup, beverage, or food item, that is swallowed within a short period of time. Thus, the subject to be treated is administered at least one unit dose per day. In some embodiments, the dosage forms can be taken in a single application or multiple applications per day. For example, if four capsules are taken daily, each capsule comprising 500 mg DHA, then all four capsules could be taken once daily, or 2 capsules could be taken twice daily, or 1 capsule could be taken every 6 hours. Various amounts of DHA can be in a unit dose. In some embodiments, the dosage form comprises less than about 10 g of DHA, less than about 5 g of DHA, about 100 mg to about 4.8 g DHA, about 200 mg to about 4.6 g of DHA, or about 500 mg to about 4.0 g DHA. In some embodiments, the unit dose comprises about 0.2 g to about 2 g DHA. In some embodiments, the unit dose comprises about 0.2 g, 0.3 g,, 0.4 g, 0.45 g, 0.50 g, 0.6 g, 0.7 g, 0.8 g, 0.9 g, 1 g, 1.1 g, 1.2 g, 1.3 g, 1.4 g, 1.5 g, 1.8 g, 2.0 g, 2.5 g, 2.7 g, 3.0 g, 3.2 g, 3.3 g, 3.4 g, 3.5 g, 3.6 g, 3.7 g, 3.8 g, 3.9 g, 4.0 g, 4.5 g, 5.0 g, 6.0 g, 6.5 g, 7 g, 8 g, 9 g, or 10 g DHA.

In some embodiments, where the DHA is in the form of an ester, preferably an alkyl ester, such as a methyl ester, ethyl ester, propyl ester, or combinations thereof, the dosage form or unit dose can comprise about 430 mg to about 480 mg of DHA ester, particularly the ethyl ester. In some embodiments, the dosage form or the unit dose can comprise about 860 mg to about 950 mg, or about 870 mg to about 930 mg of the DHA ester, particularly the ethyl ester.

In some embodiments, the unit dose has a total weight of about 0.2 to about 2 g. By way of example and not limitation, a capsule can contain a total weight of an algal oil of about 0.2 g, where the algal oil contains DHA at a certain wt% of the total fatty acid content of the oil and/or a specified wt% of the total weight of the oil. In some embodiments, the unit dose has a total weight of about 0.20 g, 0.25 g, 0.30 g, 0.35 g, 0.40 g, 0.45 g, 0.50 g, 0.55 g, 0.60 g, 0.65 g, 0.70 g, 0.75 g, 0.80 g, 0.85 g, 0.90 g, 0.95 g, 1.0 g or 1.05 g.

In some embodiments, the DHA is about 30% (w/w) or more of the total fatty acid content of the dosage form or unit dose, about 30% to about 99.9% (w/w) of the total fatty acid content of the dosage form or unit dose, about 35% to about 99.9% (w/w) of the total fatty acid content of the dosage form or unit dose, about 35% to about 60% (w/w) of the total fatty acid content of the dosage form or unit dose, about 35% to about 50% (w/w) of the total fatty acid content of the dosage form or unit dose, about 37% to about 45% (w/w) of the total fatty acid content of the dosage form or unit dose, or about 38% to about 43% (w/w) of the total fatty acid content of the dosage form or unit dose. In some embodiments, the DHA is greater than about 35%, about 37%, about 38%, about 39% or about 40% (w/w) of the total fatty acid content of the dosage form or unit dose. In some embodiments, the DHA is about 30% to about 99.5% (w/w) of the total fatty acid content of the dosage form, or about 40% to about 65% (w/w) of the total fatty acid content of the dosage form or unit dose.

In some embodiments, the DHA is greater than about 80% (w/w) of the total fatty acid content of the dosage form or unit dose, about 80% to 99.9% (w/w) of the total fatty acid content of the dosage form or unit dose, about 85% to about 99% (w/w) of the total fatty acid content of the dosage form or unit dose, about 87% to about 98% (w/w) of the total fatty acid content of the dosage form or unit dose, or about 90% to about 97% (w/w) of the total fatty acid content of the dosage form or unit dose. In some embodiments, the DHA is greater than about 95%, about 97%, about 98%, about 99% or about 99.5% (w/w) of the total fatty acid content of the dosage form or unit dose.

In some embodiments, the DHA can comprise about 40% to about 45% (w/w) of the total fatty acid content of the dosage form or unit dose. In some of embodiments, the DHA can comprise about 35% to about 45% (w/w) of the total fatty acid content of the dosage form or unit dose. In some embodiments, the DHA can comprise about 55% to about 67% (w/w) of the total fatty acid content of the dosage form or unit dose. In some embodiments, the DHA can comprise greater than about 70% (w/w) of the total fatty acid content of the dosage form or unit dose. In some embodiments, the DHA can comprise about 85% to about 99.5% (w/w) of the total fatty acid content of the dosage form or unit dose.

In some embodiments, the DHA in the dosage form or unit dose can comprise about 35% to about 96% (w/w) of the weight of the dosage form or unit dose, e.g., algal oil. In some embodiments, the DHA in the dosage form or unit dose can comprise about 38% to about 42% (w/w) of the weight of the dosage form or unit dose. In some embodiments, the DHA in the dosage form or unit dose can comprise about 35% to about 45% (w/w) of the total weight of the dosage form or unit dose. In some embodiments, the DHA in the dosage form or unit dose can comprise about 55% to about 57% (w/w) of the total weight of the dosage form or unit dose. In some embodiments, the DHA in the dosage form or unit dose can comprise about 85% to about 96% (w/w) of the total weight of the dosage form or unit dose.

As noted above, in the embodiments herein, the dosage form is substantially free of eicosapentaenoic acid (EPA). The term "EPA" and "eicosapentaenoic acid" are used interchangeably herein to refer to the compound known by its chemical name (all Z) 5,8,11,14,17-eicosapentaenoic acid, as well as any salts or derivatives thereof. Thus, the term "EPA" encompasses the free acid EPA as well as EPA alkyl esters and triglycerides containing EPA. EPA is an ω-3 polyunsaturated fatty acid. As used herein, the term "substantially free of EPA" refers to a dosage form in which EPA is less than about 3% (w/w) of the total fatty acid content of the dosage form. In some embodiments, the EPA in the dosage form comprises less than about 2% (w/w) of the total fatty acid content of the dosage form, less than 1% of the total fatty acid content of the dosage form, less than 0.5% (w/w) of the total fatty acid content of the dosage form, less than 0.2% (w/w) of the total fatty acid content of the dosage form, or less than 0.01% (w/w) of the total fatty acid content of the dosage form. In some embodiments, the dosage form has no detectable amount of EPA.

In the methods herein, the dosage form can also be substantially free of arachidonic acid (ARA). The term "ARA" and "arachidonic acid" are used interchangeably herein to refer to the compound known by its chemical name all-cis-5,8,11,14-eicosatetraenoic acid (also referred to as (5Z,8Z,11Z,14Z)-icosa-5,8,11,14-tetraenoic acid), as well as any salts or derivatives thereof. Thus, the term "ARA" encompasses the free acid ARA as well as ARA alkyl esters and triglycerides containing ARA. ARA is an ω-6 polyunsaturated fatty acid. As used herein, the term "substantially free of ARA" refers to a dosage form in which ARA is less than about 3% (w/w) of the total fatty acid content of the dosage form. In some embodiments, the ARA in the dosage form comprises, less than about 2% (w/w) of the total fatty acid content of the dosage form, less than 1% (w/w) of the total fatty acid content of the dosage form, less than 0.5% (w/w) of the total fatty acid content of the dosage form, less than 0.2% (w/w) of the total fatty acid content of the dosage form, or less than 0.01% (w/w) of the total fatty acid content of the dosage form. In some embodiments, the dosage form has no detectable amount of ARA.

In the methods herein, additional fatty acids can be present in the dosage form or unit dose. These fatty acids can include fatty acids that were not removed during the purification process, i.e., fatty acids that were co-isolated with DHA from an organism. As used herein, the term "fatty acid" includes any salts or derivatives of the fatty acid. Thus, the term "fatty acid" encompasses the free fatty acid as well as esters and triglycerides containing the fatty acid. Hence, in various embodiments, the fatty acid can be in the form of a phospholipid, a triglyceride, free fatty acid, and/or an ester. These fatty acids can be present in various concentrations. In some embodiments, the dosage form comprises 0.1% to 60% of one or more of the following fatty acids: (a) capric acid; (b) lauric acid; (c) myristic acid; (d) palmitic acid, (e) palmitoleic acid; (f) stearic acid; (g) oleic acid; (h) linoleic acid; (i) α-linolenic acid; (j) docosapentaenoic acid 22:5n-3, 22:5w3 (DPAn3); (k) docosapentaenoic acid 22:5n-6, 22:5w6 (DPAn6); and (1) 4,7,10,13,16,19,22,25 octacosaoctaenoic acid (C28:8).

In some embodiments, the dosage form comprises 20% to 40% of one or more of the following fatty acids: (a) capric acid; (b) lauric acid; (c) myristic acid; (d) palmitic acid; (e) palmitoleic acid; (f) stearic acid; (g) oleic acid; (h) linoleic acid; (i) α-linolenic acid; j) docosapentaenoic acid 22:5n-3, 22:5w3 (DPAn3); (k) docosapentaenoic acid 22:5n-6, 22:5w6 (DPAn6); and (1) 4,7,10,13,16,19,22,25 octacosaoctaenoic acid (C28:8).

In some embodiments, the dosage form or unit dose is characterized by a fatty acid content of about 0.1 % to about 20% (w/w) of one or more of the following fatty acids: (a) capric acid; (b) lauric acid; (c) myristic acid; (d) palmitic acid; (e) palmitoleic acid; (f) stearic acid; (g) oleic acid; (h) linoleic acid; (i) α-linolenic acid; (j) docosapentaenoic acid 22:5n-3, 22:5w3 (DPAn3); (k) docosapentaenoic acid 22:5n-6, 22:5w6 (DPAn6); and (1) 4,7,10,13,16,19,22,25 octacosaoctaenoic acid (C28:8).

In some embodiments, a dosage form or unit dose is characterized by a fatty acid content of about 1.0% to about 5% (w/w) of one or more of the following fatty acids: (a) capric acid; (b) lauric acid; (c) myristic acid; (d) palmitic acid; (e) palmitoleic acid; (f) stearic acid; (g) oleic acid; (h) linoleic acid; (i) α-linolenic acid; (j) docosapentaenoic acid 22:5n-3, 22:5w3 (DPAn3); (k) docosapentaenoic acid 22:5n-6, 22:5w6 (DPAn6); and (1) 4,7,10,13,16,19,22,25 octacosaoctaenoic acid (C28:8).

In some embodiments, a dosage form or unit dose is characterized by a fatty acid content of less than 1% (w/w) each of the following fatty acids: (a) capric acid; (b) lauric acid; (c) myristic acid; (d) palmitic acid; (e) palmitoleic acid; (f) stearic acid; (g) oleic acid; (h) linoleic acid; (i) α-linolenic acid; (j) docosapentaenoic acid 22:5n-3, 22:5w3 (DPAn3); (k) docosapentaenoic acid 22:5n-6, 22:5w6 (DPAn6); and (1) 4,7,10,13,16,19,22,25 octacosaoctaenoic acid (C28:8). In the descriptions herein, the term "less than" includes no detectable amount of the specified fatty acid. In some embodiments, the dosage form of described herein does not contain a measurable amount of docosapentaenoic acid 22:5n-3, 22:5w3 (DPAn3); docosapentaenoic acid 22:5n-6, 22:5w6 (DPAn6); and/or 4,7,10,13,16,19,22,25 octacosaoctaenoic acid (C28:8).

In some embodiments of the DHA dosage forms described herein, the dosage form or the unit dose is characterized by one or more the following fatty acids, expressed as wt% of the total fatty acid content. The embodiments provided herein may further comprise about 2% or less (w/w) of capric acid (C10:0). The embodiments herein may further comprise about 6% or less (w/w) of lauric acid (C12:0). The embodiments herein may further comprise about 20% or less (w/w), preferably about 5 to about 20% (w/w) of myristic acid (C14:0). The embodiments herein may further comprise about 20% or less (w/w), preferably about 5 to about 20% (w/w) of palmitic acid (C16:0). The embodiments herein may further comprise about 3% or less (w/w) of palmitoleic acid (C16:1n-7). The embodiments herein may further comprise about 2% or less (w/w) of stearic acid (C18:0). The embodiments herein may further comprise about 40% or less (w/w), preferably about 10 to about 40% (w/w) of oleic acid (C18: 1n-9). The embodiments herein may further comprise about 5% or less (w/w) of linoleic acid (C18:2). The embodiments herein may further comprise about 2% or less (w/w) of nervonic acid (C24:1). The embodiments herein may further comprise about 3% or less (w/w) of other fatty acids. The DHA dosage form or unit dose with the preceding characteristics can comprise DHASCO®, an oil derived from Crypthecodinium cohnii containing docosahexaenoic acid (DHA).

An exemplary DHA-containing oil derived from Crypthecodinium cohnii is characterized by the specified amount of components listed in Table 1, where "Max" refers to the amount of the component that can be present up to the specified amount.

**Table 1**

| **Fatty Acids** | |
|---|---|
| 10:0 | Max 2% |
| 12:0 | Max 6% |
| 14:0 | 5-20% |
| 16:0 | 5-20% |
| 16:1 | Max 3% |
| 18:0 | Max 2% |
| 18:1 | 10-40% |
| 18:2 | Max 5% |
| 22:6 DHA | 40 to 45% |
| 24:1 | Max 2% |
| Others | Max 3% |

| **Elemental Composition** | |
|---|---|
| Arsenic | Max 0.5 ppm |
| Copper | Max 0.1 ppm |
| Iron | Max 0.5 ppm |
| Lead | Max 0.2 ppm |
| Mercury | Max 0.04 ppm |
| Phosphorous | Max 10 ppm |

| **Chemcial Characteristics** | |
|---|---|
| Peroxide value | Max 5.0 meq/kg |
| Free fatty acid | Max 0.4% |
| Unsaponifiable Matter | Max 3.5% |

In some embodiments of the DHA dosage forms described herein, the dosage form or unit dose is characterized by one or more the following fatty acids, expressed as wt% of the total fatty acid content. The embodiments provided herein may further comprise about 12% or less (w/w), preferably about 6 to about 12% (w/w) of myristic acid (C14:0). The embodiments provided herein may further comprise about 28% or less(w/w), preferably about 18 to about 28% (w/w) of palmitic acid (C16:0). The embodiments provided herein may further comprise about 2% or less (w/w) of stearic acid (C18:0). The embodiments provided herein may further comprise about 8% or less (w/w) of oleic acid (C18:1n-9). The embodiments provided herein may further comprise about 2% or less (w/w) of linoleic acid (C18:2). The embodiments provided herein may further comprise about 2% or less (w/w) of arachidonic acid (C20:4). The embodiments provided herein may further comprise about 3% or less (w/w) of eicosapentaenoic acid (C20:5). The embodiments provided herein may further comprise about 18% or less (w/w), preferably about 12 to about 18% (w/w) of docosapentaenoic acid (22:5n-6). The embodiments provided herein may further comprise about 10% or less (w/w) of other fatty acids. In some of these embodiments, the ratio of wt% of DHA to wt% of DPAn6 is about 2.5 to about 2.7. The DHA dosage form or unit dose with the preceding characteristics can comprise Life's DHA™ (also formerly referenced as DHA™-S and DHASCO®-S), an oil derived from the Thraustochytrid, Schizochytrium sp., that contains a high amount of DHA and also contains docosapentaenoic acid (n-6) (DPAn-6).

An exemplary DHA-containing oil derived from Schizochytrium sp. is characterized by the specified amount of components listed in Table 2, where "Max" refers to the amount of the component that can be present up to the specified amount.

**Table 2**

| **Fatty Acids** | |
|---|---|
| 14:0 | 6-12.0% |
| 16:0 | 18-28% |
| 18:0 | Max 2% |
| 18:1 | Max 8% |
| 18:2 | Max 2% |
| 20:4 ARA | Max 2% |
| 20:5 EPA | Max 3% |
| 22:5n-6 DPA | 12-18% |
| 22:6 DHA | Min 35% |
| Others | Max 10% |

| **Elemental Composition** | |
|---|---|
| Arsenic | Max 0.2 ppm |
| Copper | Max 0.05 ppm |
| Iron | Max 0.2 ppm |
| Lead | Max 0.1 ppm |
| Mercury | Max 0.04 ppm |

| **Chemcial Characteristics** | |
|---|---|
| Peroxide value | Max 5.0 meq/g |
| Free fatty acid | Max 0.25% |
| Moisture and Volatiles | Max 0.05% |
| Unsaponifiable Matter | Max 4.5% |
| Trans fatty acids | Max 1% |

In some embodiments of the DHA dosage forms described herein, the dosage form or unit dose is characterized by one or more the following fatty acids, expressed as wt% of the total fatty acid content. The embodiments provided herein may further comprise about 2% or less (w/w) of capric acid (C10:0). The embodiments provided herein may further comprise about 6% or less (w/w) of lauric acid (C12:0). The embodiments provided herein may further comprise about 20% or less (w/w), preferably about 10 to about 20% (w/w) of myristic acid (C14:0). The embodiments provided herein may further comprise about 15% or less (w/w), preferably about 5 to about 15% (w/w) of palmitic acid (C16:0). The embodiments provided herein may further comprise about 5% or less (w/w) of palmitoleic acid (C16: In-7). The embodiments provided herein may further comprise about 2% or less (w/w) of stearic acid (C18:0). The embodiments provided herein may further comprise about 20% or less (w/w), preferably about 5% to about 20% of oleic acid (C18:1n-9). The embodiments provided herein may further comprise about 2% or less of linoleic acid (C18:2). The embodiments provided herein may further comprise about 2% or less of nervonic acid (C24:1). The embodiments provided herein may further comprise about 3% or less of other fatty acids. The DHA dosage form or unit dose with the preceding characteristics may be an oil derived from Crypthecodinium cohnii containing docosahexaenoic acid (DHA).

An exemplary DHA-containing oil derived from Crypthecodinium cohnii is characterized by the specified amount of components listed in Table 3, where "Max" refers to the amount of the component that can be present up to the specified amount..

**Table 3**

| **Fatty Acids** | |
|---|---|
| 10:0 | 0-2% |
| 12:0 | 0-6% |
| 14:0 | 10-20% |
| 16:0 | 5-15% |
| 16:1 | 0-5% |
| 18:0 | 0-2% |
| 18:1 | 5-20% |
| 18:2 | 0-2%% |
| 22:6 n-3 DHA | 57-65% |
| 24:1 | 0-2% |
| Others | 0-3% |

| **Elemental Composition** | |
|---|---|
| Arsenic | Max 0.5 ppm |
| Copper | Max 0.1 ppm |
| Iron | Max 0.5 ppm |
| Lead | Max 0.2 ppm |
| Mercury | Max 0.2 ppm |
| Phosphorous | Max 10 ppm |

| **Chemical Characteristics** | |
|---|---|
| Peroxide value | Max 5.0 meq/kg |
| Free fatty acid | Max 0.4% |
| Unsaponifiable Matter | Max 3.5% |
| Trans fatty acids | <3.5% |
| Moisture and Volatiles | <0.1% |
| Insoluble impurities | <0.1% |

In some embodiments, the DHA in the dosage form or unit dose comprises a ratio of wt% of DHA to wt% DPAn6 of about 2.5 to about 2.7. In some of these embodiments, the DHA in the dosage form or unit dose comprises greater than about 70% (w/w) of the total fatty acid content of the dosage form or unit dose. The DHA in dosage form or unit dose with the preceding characteristics may be in the form of a DHA ester, preferably an alkyl ester, such as a methyl ester, ethyl ester, propyl ester, or combinations thereof, derived from an algal oil from the Thraustochytrid, Schizochytrium sp.

In some embodiments of the DHA dosage forms described herein, the dosage form or unit dose is characterized by one or more the following fatty acids, expressed as wt% of the total fatty acid content. The embodiments provided herein may further comprise about 0.1% or less (w/w) to undetectable levels of myristic acid (C14:0). The embodiments provided herein may further comprise about 0.5% or less (w/w) of palmitic acid (C16:0). The embodiments provided herein may further comprise about 0.5% or less (w/w) of palmitoleic acid (C16: In-7). The embodiments provided herein may further comprise about 0.5% or less (w/w), or undetectable levels of stearic acid (C18:0). The embodiments provided herein may further comprise about 4% or less (w/w) of oleic acid(C18:1n-9). The embodiments provided herein may further comprise less than 0.1% (w/w) or undetectable levels of linoleic acid (C18:2). The embodiments provided herein may further comprise less than 0.1% (w/w), or undetectable levels of eicosapentaenoic acid (C20:5). The embodiments provided herein may further comprise about 2% or less (w/w) of docosapentaenoic acid (22:5n-3). The embodiments provided herein may further comprise about 1% or less (w/w) of octacosaoctaenoic acid (28:8n-3). The embodiments provided herein may further comprise about 0.5% or less (w/w) of tetracosaenoic acid (24:1ω9). The embodiments provided herein may further comprise about 1% or less (w/w) of other fatty acids. The DHA in dosage form or unit dose with the preceding characteristics may be in the form of a DHA ester, preferably an alkyl ester, such as a methyl ester, ethyl ester, propyl ester, or combinations thereof, derived from an algal oil from Crypthecodinium cohnii.

A DHA-containing oil derived from the algal oil of Crypthecodinium cohnii, where the DHA comprises an ethyl ester, is characterized by the specified amount of components listed in **Table 4.**

**Table 4**

| **DHA content (mg/g)** | |
|---|---|
| 855-945 | |

| **Fatty Acid Content: % of total EE** | |
|---|---|
| Eicosapentaenoic Acid (20:5ω3) | ND |
| Myristic Acid (14:0) | 0.1% |
| Palmitic Acid (16:0) | 0.5% |
| Palmitoleic Acid (16:1ω7) | 0.4% |
| Stearic Acid (18:0) | ND |
| Oleic Acid (18:1ω9) | 4% |
| Linoleic Acid (18:2ω6) | ND |
| Docosapentaenoic acid (22:5ω3) | 1.3% |
| Octacosaoctaenoic acid (28:8ω3) | 0.9% |
| Tetracosaenoic Acid (24:1ω9) | 0.3% |
| Others | 1.1% |

| **Elemental Composition** | |
|---|---|
| Arsenic | Max 0.5 ppm |
| Copper | Max 0.1 ppm |
| Iron | Max 0.5 ppm |
| Lead | Max 0.2 ppm |
| Mercury | Max 0.04 ppm |

| **Chemical Characteristics** | |
|---|---|
| Peroxide value | Max 10.0 meq/kg |

| | |
|---|---|
| ND = not detectable | |

In some embodiments, the DHA esters can be derived from undiluted oil from a single cell microorganism, and in some embodiments, from undiluted DHASCO-T^{®} (Martek Biosciences Corporation). In some embodiments, the oil from which DHA are derived include single cell microorganism oils that are manufactured by a controlled fermentation process followed by oil extraction and purification using methods common to the vegetable oil industry. In certain embodiments, the oil extraction and purification steps include refining, bleaching, and deodorizing. In some embodiments, the undiluted DHA oil contains about 40% to about 50% DHA by weight (about 400-500 mg DHA/g oil). In certain embodiments, the undiluted DHA oil is enriched by cold fractionation (resulting in oil containing about 60% w/w of DHA triglyceride), which DHA fraction optionally can be transesterified, and subjected to further downstream processing to produce the active DHA. In some embodiments, downstream processing of the oil can comprises distillation and/or silica refinement.

Accordingly, to produce oil form which the DHA is derived, in certain aspects, the following steps can be used: fermentation of a DHA producing microorganism; harvesting the biomass; spray drying the biomass; extracting oil from the biomass; refining the oil; bleaching the oil; chill filtering the oil; deodorizing the oil; and adding an antioxidant to the oil. In some embodiments, the microorganism culture is progressively transferred from smaller scale fermenters to a production size fermenter. In some embodiments, following a controlled growth over a pre-established period, the culture is harvested by centrifugation then pasteurized and spray dried. In certain embodiments, the dried biomass is flushed with nitrogen and packaged before being stored frozen at -20°C. In certain embodiments, the DHA oil is extracted from the dried biomass by mixing the biomass with n-hexane or isohexane in a batch process which disrupts the cells and allows the oil and cellular debris to be separated. In certain embodiments, the solvent is then removed.

In some embodiments, the crude DHA oil then undergoes a refining process to remove free fatty acids and phospholipids. The refined DHA oil is transferred to a vacuum bleaching vessel to assist in removing any remaining polar compounds and pro-oxidant metals, and to break down lipid oxidation products. The refined and bleached DHA oil undergoes a final clarification step by chilling and filtering the oil to facilitate the removal of any remaining insoluble fats, waxes, and solids.

Optionally, the DHA is deodorized under vacuum in a packed column, counter current steam stripping deodorizer. Antioxidants such as ascorbyl palmitate and alpha-tocopherol can optionally be added to the deodorized oil to help stabilize the oil. In some embodiments, the final, undiluted DHA oil is maintained frozen at -20°C until further processing. An exemplary undiluted DHA oil is characterized by amount of components listed in Table 5, where "Max" refers to the amount of the component that can be present up to the specified amount..

**Table 5: Characteristics of Undiluted DHA Oil**

| **Component** | **Specification** |
|---|---|
| DHA content mg/DHA/g oil | 480 mg/g |
| Free Fatty Acid | Max.0.4% |
| Peroxide Value (PV) | Max. 5 meq/kg |
| Anisidine Value (AV) | Max 20 |
| Moisture and Volatiles (M & V) | Max. 0.02% |
| Unsaponifiable Matter | Max. 3.5% |
| Insoluble Impurities | Max. 0.1% |
| Trans Fatty Acid | Max. 1% |
| Arsenic | Max. 0.5 ppm |
| Cadmium | Max. 0.2 ppm |
| Chromium | Max. 0.2 ppm |
| Copper | Max. 0.1 ppm |
| Iron | Max. 0.5 ppm |
| Lead | Max. 0.2 ppm |
| Manganese | Max. 0.04 ppm |
| Mercury | Max. 0.04 ppm |
| Molybdenum | Max. 0.2 ppm |
| Nickel | Max.0.2 ppm |
| Phosphorus | Max.10 ppm |
| Silicon | Max. 500 ppm |
| Sulfur | Max. 100 ppm |
| 18:1 n-9 Oleic Acid | Max. 10% |
| 20:5 n-3 EPA | Max. 0.1% |
| Unknown Fatty Acids | Max. 3.0% |

In some embodiments, the DHA oil is converted to DHA ester by methods known in the art. In some embodiments, DHA ester can be produced from DHA oil by the following steps: cold fractionation and filtration of the DHA oil (to yield for example about 60% triglyceride oil); direct transesterification (to yield about 60% DHA ethyl ester); molecular distillation (to yield about 88% DHA ethyl ester); silica refinement (to yield about 90% DHA ethyl ester); and addition of an antioxidant.

In some embodiments, the cold fractionation step is carried out as follows: undiluted DHA oil (triglyceride) at about 5000 mg/g DHA is mixed with acetone and cooled at a controlled rate in a tank with -80°C chilling capabilities. Saturated triglycerides crystallize out of solution, while polyunsaturated triglycerides at about 600 mg/g DHA remain in the liquid state. The solids containing about 300 mg/g are filtered out with a 20 micron stainless steel screen from the liquid stream containing about 600 mg/g DHA. The solids stream is then heated (melted) and collected. The 600 mg/g DHA liquid stream is desolventized with heat and vacuum and then transferred to the transesterification reactor.

In some embodiments, the transesterification step is carried out on the 600 mg/g DHA oil, wherein the transesterification is done via direct transesterification using ethanol and sodium ethoxide. The transesterified material (DHA-EE) is then subject to molecular distillation and thus, further distilled (3 passes, heavies, lights, heavies) to remove most of the other saturated fatty acids and some sterols and non-saponifiable material. The DHA-EE is further refined by passing it through a silica column.

In some embodiments herein, the dosage form is a pharmaceutical dosage form. "Pharmaceutically acceptable" refers to compositions that are, within the scope of sound medical judgment, suitable for contact with the tissues of human beings and animals without excessive toxicity or other complications commensurate with a reasonable benefit/risk ratio. In some embodiments, the compounds (e.g., DHA), compositions, and dosage forms herein are pharmaceutically acceptable.

In some embodiments, the dosage form is a nutraceutical dosage form. The term "nutraceutical" refers to any substance that is (1) a sole item of a meal or diet that provides medical and/or health benefits, or (2) a product that is intended to supplement the diet that bears or contains one or more of the following dietary ingredients: a vitamin, a mineral, an herb or other botanical, an amino acid, a dietary substance for use by man to supplement the diet by increasing the total daily intake, or a concentrate, metabolite, constituent, extract, or combinations of these ingredients that provides medical and/or health benefits. The medical and/or health benefits can include reducing the risk of a condition by increasing cognitive function and/or decreasing heart rate. For example, in some embodiments, normal aging decreases a subject's ability to remember and learn. DHA can ameliorate, improve and/or augment the ability to learn and to remember as one ages.

The DHA can be formulated in a dosage form. These dosage forms can include, but are not limited to, tablets, capsules, cachets, pellets, pills, gel caps, powders and granules; and parenteral dosage forms which include, but are not limited to, solutions, suspensions, emulsions, coated particles, and dry powder comprising an effective amount of the DHA as described herein. In some embodiments, the dosage form can be inserted or mixed into a food substance. Various substances are known in the art to coat particles, including cellulose derivatives, e.g., microcrystalline cellulose, methyl cellulose, carboxymethyl cellulose; polyalkylene glycol derivatives, e.g., polyethylene glycol; talc, starch, methacrylates, etc. In some embodiments, the dosage form is a capsule, wherein the capsule is filled with a solution, suspension, or emulsion comprising the DHA It is also known in the art that the active ingredients can be contained in such formulations with pharmaceutically acceptable excipients such as diluents, fillers, disintegrants, binders, lubricants, surfactants, hydrophobic vehicles, water soluble vehicles, emulsifiers, buffers, humectants, moisturizers, solubilizers, preservatives, flavorants, taste-masking agents, sweeteners, and the like. Suitable excipients can include, e.g., vegetable oils (e.g., com, soy, safflower, sunflower, or canola oil). In some embodiments, the preservative can be an antioxidant, e.g., sodium sulfite, potassium sulfite, metabisulfite, bisulfites, thiosulfates, thioglycerol, thiosorbitol, cysteine hydrochloride, butylated hydroxytoluene, butylated hydroxyanisole (BHA); ascorbic acid and derivatives thereof, such as ascorbyl palmitate; gallates, such as propyl gallate and gallic acid; quinine; tocopherol, such as α-, β-, γ-, and δ-tocopherols; carotenoids. such as lutein, lycopene, and beta-carotene; and combinations thereof. The means and methods of making the dosage forms for administration are known in the art, and an artisan can refer to various pharmacologic references for guidance. For example, "Modern Pharmaceutics," Banker & Rhodes, Informa Healthcare, 4th ed. (2002); "Goodman & Gilman's The Pharmaceutical Basis of Therapeutics," McGraw-Hill, New York, 10th ed. (2001); and Remingtons's Pharmaceutical Sciences, 20th Ed., 2001 can be consulted.

The DHA of the present invention is orally active and this route of administration can be used in the methods described herein. Accordingly, administration forms can include, but are not limited to, tablets, dragees, capsules, caplets, gelatin capsules, and pills, which contain the DHA and one or more suitable pharmaceutically acceptable carriers.

For oral administration, the DHA can be administered as an oil or it can be formulated readily by combining it with a pharmaceutically acceptable carrier or with pharmaceutically acceptable carriers. Pharmaceutical acceptable carriers are well known in the art Such carriers enable the compounds to be formulated as tablets, gel caps, pills, dragees, capsules, liquids, gels, syrups, slurries, suspensions and the like, for oral ingestion by a subject to be treated. In some embodiments, the dosage form is a tablet, gel cap, pill or caplet. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl cellulose, sodium carboxymethyl cellulose, vegetable oil (e.g., soybean oil), and polyvinylpyrrolidone (PVP). If desired, disintegrating agents can be added, such as, but not limited to, the cross-linked polyvinyl pyrrolidone, agar, or alginic acid or a salt thereof such as sodium alginate. Pharmaceutical preparations which can be used orally include, but are not limited to, push-fit capsules made of gelatin, as well as soft, sealed capsules made of gelatin and a plasticizer, such as glycerol or sorbitol. Capsule shells can be composed of non-animal derived ingredients, i.e., vegetarian ingredients, such as carrageenan, alginate, modified forms of starch, cellulose and/or other polysaccharides. Gelatin capsules comprising DHA are described in US application Ser. No. 61/247,944, entitled "Docosahexaenoic Acid Gel Caps," filed October 1, 2009, the contents of which is incorporated herein by reference. All formulations for oral administration should be in dosages suitable for such administration.

As noted above, in some embodiments, the dose may be provided as a dietary supplement or as a medical food.

An exemplary gel capsule is a soft gelatin capsule of about 1 g, having specifications within the limits set forth in Table 6:

**Table 6: Specifications for 1 gram DHA Ethyl Ester Gel Capsules**

| **Test** | **Specification** |
|---|---|
| DHA content, mg DHA/g oil | 855 - 945 mg/g |
| Ethyl Ester | Min. 90% esterified |
| Acid Value | Max. 2.0 mg KOH/g |
| Peroxide Value (PV) | Max. 10 meq/kg |
| Anisidine Value (AV) | Max. 20 |
| Trans Fatty Acid | Max. 2.0% |
| Arsenic | Max. 0.5 ppm |
| Copper | Max. 0.1 ppm |
| Iron | Max. 0.5 ppm |
| Lead | Max. 0.2 ppm |
| Mercury | Max. 0.04 ppm |
| E. coli | Absent in 1 g |
| Total Plate Count | <1000 cfu/g |
| Yeast and Molds | <100 cfu/g |

In some embodiments, the gel capsule comprises a capsule preparation, an active, and optionally a colorant and/or antioxidant In another embodiment i) the capsule preparation comprises gelatin (bovine acid hide), glycerin, and purified water, ii) the active comprises DHA-EE, iii) the optional colorant is selected from titanium dioxide, FD&C Yellow #5, FD&C Red 40, and mixtures thereof; and iv) the antioxidant is ascorbyl palmitate. In some embodiments, the raw materials are USP raw materials.

An exemplary gel capsule is a soft gelatin capsule of about 1 g, having the specifications within the limits set forth in **Table 7:**

**Table 7: Specifications for 1 gram DHA Ethyl Ester Gel Capsules**

| **Test** | **Specification** |
|---|---|
| DHA EE Content, per capsule | 855 - 945 mg |
| Average Fill Weight | 950 -1050 mg |
| Disintegration | Complies USP |
| Acid Value | Max. 2.0 mg KOH/g |
| Peroxide Value (PV) | Max.10 meq/kg |
| Anisidine Value (AV) | Max. 20 |
| Microbial Limits Tests | Complies with <61> USP |

Set forth in **Table 8** is an exemplary list of components that are used in the manufacture of a DHA ethyl ester soft gelatin capsule, and at least one corresponding function for each component.

**Table 8: List of Components in 1 gram DHA Ethyl Ester Soft Gelatin Capsules**

| **Component** | **Function** |
|---|---|
| 900 mg DHA EE | Active |
| Gelatin, Bovine Acid Hide | Capsule Preparation |
| Glycerin | Capsule Preparation |
| Purified Water | Capsule Preparation |
| Titanium Dioxide | Colorant |
| FD&C Yellow #5 | Colorant |
| FD&C Red #40 | Colorant |

As described herein, in some embodiments, the present invention is directed to methods of improving cognitive function in a subject A decline in memory and cognitive function is considered to be a normal consequence of aging in humans. Age-related cognitive decline (ARD), or "age-associated memory impairment" (AAMI), is a term used to describe "older persons with objective memory declines relative to their younger years, but cognitive functioning that is normal relative to their age peers." See e.g., APA Presidential Task Force on the Assessment of Age-Consistent Memory Decline and Dementia, February 1998. In some embodiments, ARCD is defined as an objectively identified decline in cognitive functioning consequent to the aging process that is within normal limits given a person's age. Individuals may report problems remembering names or appointments or may experience difficulty solving complex problems." Diagnostic and Statistical Manual of Mental Disorders. Fourth ed. Washington, DC: American Psychiatric Association; 1994. Age-related cognitive decline is different from Mild Cognitive Impairment (MCI) "which is more severe or consistent... and may indicate the early stages of a condition such as dementia." See e.g., Memory Disorders Project, Rutgers University (world wide web at memorylossonline.com/glossary/aami.html). MCI includes memory impairment in a non-demented subject beyond that expected for age and education. In some embodiments, MCI can be diagnosed in a subject as determined by Petersen et al, Arch. Neurol. 56:303-308 (1999), including the criteria of (1) memory complaint, (2) normal activities of daily living, (3) normal general cognitive function, (4) abnormal memory for age, and (5) not demented. In some embodiments, the invention is directed to improving cognitive function in a subject having age-related cognitive decline, who does not suffer from, or been diagnosed with mild cognitive impairment, Alzheimer's disease, dementia, vascular dementia, mixed dementia, dementia with Lewy Bodies, dementia caused by drugs, delirium, depression, or combinations thereof.

In some embodiments, MCI is associated with increased levels of expression or dysfunction of amyloid β (Aβ), tau protein, presenilin-1 (PS1) protein, or combinations thereof in a subject. In some embodiments, the amyloid β (Aβ), tau protein, presenilin-1 (PS 1) protein, or combinations thereof are isolated from cerebral spinal fluid and/or from plasma. Therefore, in some embodiments, the present invention is directed to a method of improving cognitive function in a subject having age-related cognitive decline, wherein the subject does not have a detectable relative increased level of expression or dysfunction of amyloid β (Aβ), tau protein, presenilin-1 (PS1) protein, or combinations thereof. In some embodiments, the level of expression or dysfunction of amyloid β (Aβ), tau protein, or presenilin-1 (PS1) protein is similar to that of a subject having AD.

In some embodiments, the invention is directed to improving cognitive decline in a subject having mild cognitive impairment. In some embodiments, the cause of the cognitive decline may be unknown and/or difficult (or impossible) to diagnose, or the cognitive decline can be the result of a combination of ARCD and MCI. Thus, the methods herein can be directed to improving cognitive decline in a subject having mild cognitive impairment and age-related cognitive decline.

In some embodiments, the method is directed to reducing the heart rate in a subject, the method comprising administering to the subject in need thereof a dosage form comprising docosahexaenoic acid substantially free of EPA. The term "heart rate" refers the average resting heartbeat per minute. When resting, the average adult human heart beats at about 60 to 80 beats per minute (bpm) in humans. This rate varies among different people, and can be significantly lower in individuals who participate in endurance athletics. Thus, as defined herein, the term "reduced heart rate" refers to a relative reduction in heart rate of a subject before and after administration of the DHA. In some embodiments, the reduction in heart rate is determined after 1 month, 2 months, 3 months, 6 months, or 1 year of administration of the DHA of the present invention. Heart rate can be determine by one of skill in the art, and can include manual determination, a stethoscope, and auscultation. Some studies have identified resting heart rate as an independent risk factor for cardiovascular disease. See, e.g., Cook, S., et al., EurHeartJ27:2387-2393 (2006). Additional, heart rate lowering is associated with reduction in cardiac deaths in post-myocardial infarction patients. See, e.g., Cucherat, M., et al., Eur Heart J27:590 (2006). In some embodiments, the method herein can be used to reduce hypertension. While not being bound by a particular theory, the reduced heart rate as well as the reduced blood pressure can decrease the cardiac load, thereby reducing the amount of work the heart must perform. Thus, the method described herein can contribute to overall cardiovascular health. While not being bound by any particular theory, in some embodiments, the method of the present invention normalizes (a) the transient outward current, (b) the delayed rectifier current, (c) Ca²⁺-Mg²⁺-ATPase, (d) Na⁺-Ca²⁺-exchanger, (e) Ca²⁺ uptake in the sarcoplasmic reticulum, (f) L-type Ca²⁺ current, or (g) combinations thereof In some embodiments, the method of the present invention prevents Ca²⁺ overload in cytoplasm and mitochondria, and or reduces the activity of membrane phospholipases responsible for the generation of intracellular messengers for Ca²⁺ handling, e.g., diacylglycerol and IP₃.

In some embodiments, the heart rate is determined at a resting state. In some embodiments, the heart rate is determined at the "maximum heart rate" (MHR, also called STD, or HRₘₐₓ), which is the highest number of times the heart can contract in one minute in a subject, or the heart rate that the subject could achieve during maximal physical exertion. In some embodiments, the heart rate is determined at the "recovery heart rate." This is the heart rate measured at a fixed (or reference) period after ceasing activity, typically measured over a 1 minute period.

In some embodiments of the present invention, the heart rate is reduced about 1 to 10 beats per minute, or about 1 to about 5 beats per minute, or about 2 to about 4 beats per minute in the subject after administration of the DHA regimen for about 1 month, 2 months, 3 months, 6 months or 1 year.

In some embodiments, the subject chosen for treatment to reduce heart rate can also have mild cognitive impairment or age related cognitive decline. In such embodiments, the cognitive disorders can be treated in addition to the reduction in heart rate. Thus, in some embodiments, the method for treating a subject to reduce the heart rate can further comprise the step of selecting the subject in need of reduction in heart rate. The selection can be made based on the criteria described herein for assessing cognitive disorders and assessing patients for treatment to reduce their heart rate.

The method of the present invention is also directed to reducing the risk of a decline in cognitive function associated with normal aging. Thus, in some embodiments the method can prevent cognitive decline in a subject. In some embodiments, the method can reduce the risk of memory loss associated with age-related cognitive decline, and/or maintain healthy (normal) cognitive function in a subject. In some embodiments, the method is directed to improving or promoting memory function in a subject who already exhibits symptoms associated with age-related cognitive decline.

The method of the present invention is also directed to improving an older subject's performance on a memory test and/or an executive function measure, e.g., a subject over the age of 50 years old. In some embodiments, the method improves problem-solving skills, planning skills, ability to concentrate, ability to pay attention, and/or reaction time in a subject. In some embodiments, the method of the present invention promotes healthy vision and/or healthy eyesight in a subject. The method can also improve daily living skills in a subject, or improve a subject's ability to perform daily tasks.

The method of the present invention can also be directed to improving cognitive function associated as measured by various secondary measurements, e.g., CANTAB VRM, PRM, SOC, SWM, MMSE and/or Geriatric Depression tests. Each of these cognitive measures are further described herein, but some refer to computerized neuropsychological tests, e.g., CANTAB®, developed by Cambridge Cognition Ltd. (Cambridge, England). Mini-mental State Examination (MMSE) is a brief, quantitative measure of cognitive status in adults (Folstein et al., Pyschiatr. Res. 12:189-198 (1975) and available from Psychological Assessment Resources (PAR) Inc. (Lutz, FL). Thus, in some embodiments, the invention is directed to improving one or more secondary measurements, e.g., VRM scores, in a subject. In some embodiments, the method is directed to improving cognitive functions, e.g., memory functions, in an individual, wherein the subject suffers from early stage cognitive decline, or has not been diagnosed with any cognitive decline.

In some embodiments, the cognitive function is measured by determining CANTAB cognitive battery including working memory, memory retention, attention, and executive function assays. In some embodiments, the cognitive function improved is measured by the number of errors made on the PAL 6 pattern stage. In some embodiments, the cognitive function improved is measured by the delayed logical memory test of the WMSIII, the CANTAB Verbal Recognition Memory test, the CANTAB Pattern Recognition test, the CANTAB Spatial Working memory test, Stockings of Cambridge test, or combinations thereof.

The DHA can be administered to a subject having a logical memory baseline score ≥ 1 standard deviation below a mean of a younger age group. In some embodiments, the subject is 50 years old or older, or 68 years old or older.

In some embodiments, various factors can play a role in the efficacy of DHA administered in the method herein. For example, a family history of dementia can be used as a predictive indicator of increased efficacy of DHA in the method of the present invention. Thus, in some embodiments, the invention is directed to a method of improving cognitive function and/or decreasing heart rate in a subject having a family history of dementia by administering a dosage form comprising docosahexaenoic acid (DHA) substantially free of eicosapentaenoic acid (EPA). In some embodiments, concomitant statin use can be used as a predictive indicator of increased efficacy of DHA in the method of the present invention. Thus, in some embodiments, the invention is directed to a method of improving cognitive function and/or decreasing heart rate in a subject who is concomitantly administered a statin, by administering a dosage form comprising docosahexaenoic acid (DHA) substantially free of eicosapentaenoic acid (EPA).

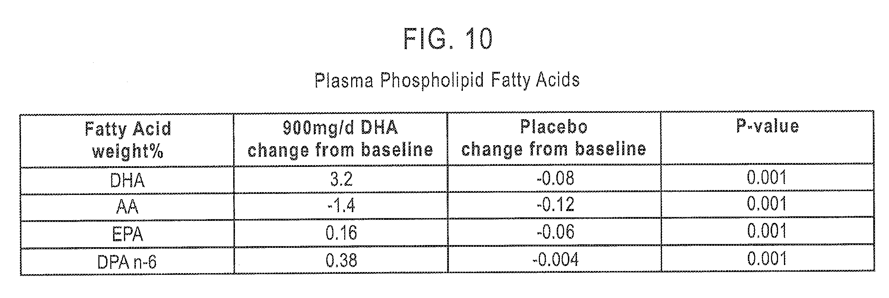

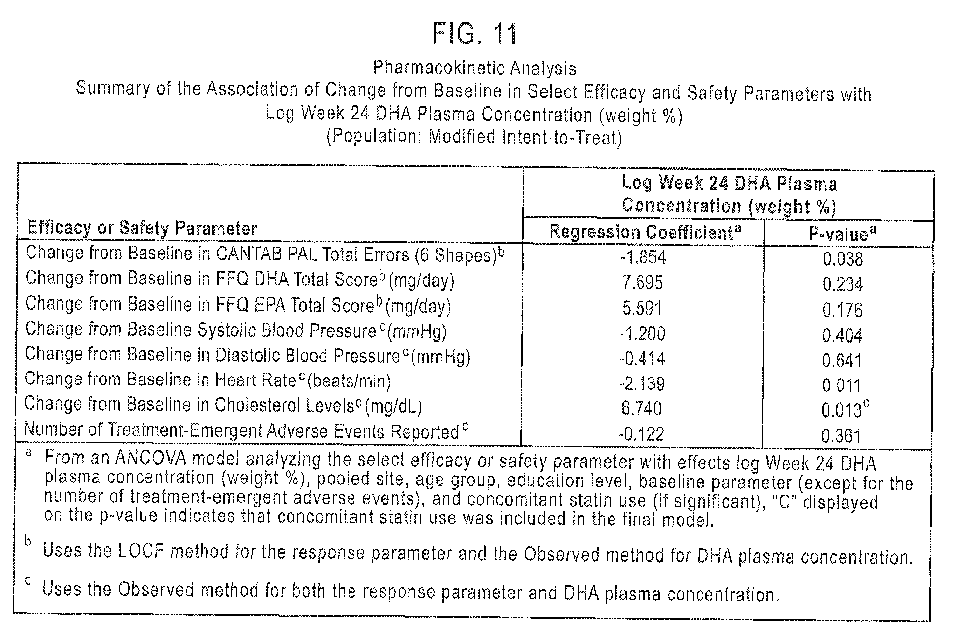

In some embodiments, the present invention is directed to a method of increasing plasma phospholipid DHA levels in a subject, increasing the plasma phospholipid DPAn-6 levels in a subject, and/or decreasing the plasma phospholipid arachidonic acid (ARA) levels in a subject. While not being bound by a particular theory, in some embodiments, the alteration of one or more phospholipid levels results in improvement of cognitive function and/or decreasing heart rate. This is shown in FIGS. 10 and 11 with r=-2.14 (p<0.011) demonstrating that as plasma DHA levels increase, change from baseline in heart rate goes down in older adults with age-related cognitive decline.

Cognitive function can be determined by various means as described herein and are known to one of skill in the art. See, e.g., the methods as outlined in Example 1. The cognitive function in a subject can be increased relative to a subject that has not been administered a dosage form comprising DHA. In some embodiments of the present invention, the cognitive function as measured by any of the techniques listed above can be increased greater than 5%, or about 5% to about 90%, about 10% to about 80%, about 25% to about 75%, or about 30% to about 65% as measured by one of the assays described herein. In some embodiments, the cognitive function in a subject can be increased relative to a subject that has been administered a dosage form comprising DHA and EPA, e.g., Lovaza®. In these instances, the cognitive function as measured by any of the techniques above can be increased greater than 5%, or about 5% to about 90%, about 10% to about 80%, about 25% to about 75%, or about 30% to about 65% relative to a subject administered DHA and EPA. One of skill in the art will appreciate that the amount of the increase can be dependent on various parameters, such as the initial cognitive function of the subject. For example, in subjects having a reduced cognitive function, the amount of the increase in cognitive function can be greater, relative to a subject with average cognitive function. The increase in cognitive function can also be dependent on the length and/or amount of administration of DHA, or the regimen of administration of the DHA. For example, in some embodiments, the cognitive decline in the subject is chronic, and thus the DHA is administered for the remainder of the subject's lifetime, or from 1 to 20 years, or 1, 2, 5, 10, or 15 years. In some embodiments, the cognitive function in a subject is increased by any of the measures listed herein by greater than 5%, about 5% to about 90%, about 25% to about 75%, or about 30% to about 65% by year 1, 5, 10, 15 or 20 years.

In some embodiments, the increase in cognitive function is determined by comparing the cognitive function of the subject being administered to the DHA to a subject of approximately the same age and physical condition, i.e., a peer. In some embodiments, the increase in cognitive function is determined by comparing the cognitive function of the individual before and after being administered DHA for 1 month, 2 months, 3 months, 6 months, 1 year or 5 years, according to the invention for subject being administered the DHA.

In some embodiments, administration of the DHA of the present invention increases cognitive function in a relatively short duration, e.g., 1 week to 26 weeks (week 1 to week 26). In some embodiments, the cognitive function in a subject is increased by greater than 5%, about 5% to about 90%, about 25% to about 75%, or about 30% to about 65% on week 26. In some embodiments, the DHA is administered daily for 1 week to 6 weeks (week 1 to week 6). In some embodiments, the cognitive function in a subject is increased by greater than 5%, about 5% to about 90%, about 25% to about 75%, or about 30% to about 65% on week 6. In some embodiments, the DHA is administered daily for 2 weeks to 4 weeks (week 2 to week 6). In some embodiments, the cognitive function in a subject is increased by greater than 5%, about 5% to about 90%, about 25% to about 75%, or about 30% to about 65% on week 6. In some embodiments, the DHA is administered daily for 28 days (day 28). In some embodiments, cognitive function in a subject is increased by greater than 5%, about 5% to about 90%, about 25% to about 75%, or about 30% to about 65% by day 28.

As used herein, the terms "treat" and "treatment" refer to both therapeutic treatment and prophylactic or preventative measures, wherein the object is to prevent or slow down (lessen) an undesired physiological condition, disorder or disease, or obtain beneficial or desired clinical results. For purposes herein, beneficial or desired clinical results include, but are not limited to, alleviation of symptoms associated with reduced cognitive function, i.e., cognitive aging; diminishment of the extent of the condition associated with reduced cognitive function; stabilization (i.e., not worsening) of the state of the condition, disorder or disease associated with reduced cognitive function; delay in onset or slowing of the condition, disorder or disease progression associated with reduced cognitive function; amelioration of the condition, disorder or disease state, remission (whether partial or total) of the condition, disorder or disease associated with reduced cognitive function, whether detectable or undetectable; or augmentation, mitigation, enhancement or improvement of the condition, disorder or disease associated with reduced cognitive function. Where the treatment is for reduction in heart rate, the beneficial or desired clinical results include, but are not limited to, reduction in cardiovascular disease risk, reduction in hypertension (lower blood pressure), reduction in incidence of myocardial ischaemia, and/or increased cardiovascular output. Treatment includes eliciting a clinically significant response, without excessive levels of side effects.

Dementia, Alzheimer's disease, mild cognitive impairment, age-related cognitive decline or age-associated memory impairment, and unexplained memory loss can all be associated with reduced cognitive function. Thus, in some embodiments, the invention is directed to methods of reducing, preventing, or slowing the development of dementia, Alzheimer's disease, mild cognitive impairment, age-related cognitive decline or age-associated memory impairment, prodromal Alzheimer's disease, and unexplained memory loss comprising administration of the DHA dosages of the present invention. The term "preventing" can mean to stop or hinder a disease, disorder, or symptom of a disease or condition that would result in cognitive decline. Thus, the phrase "preventing a decrease in cognitive function in a subject having age related cognitive decline" would include stopping or hindering the progression of cognitive decline that would naturally occur in an individual as he/she ages.

The term "subject" refers to mammals such as humans or primates, such as apes, monkeys, orangutans, baboons, gibbons, and chimpanzees. The subject can be a human or non-human. The subject can be of any age. For example, in some embodiments, the subject is a human greater than about 50 years old, greater than about 55 yrs old, greater than about 60 yrs old, greater than about 65 yrs old, greater than about 68 yrs old, greater than about 70 yrs old, greater than about 72 yrs old, or greater than about 75 years old. In some embodiments, the subject is an adult, either male or female.

In some embodiments, the subject is a "subject in need thereof." A subject in need thereof refers to an individual for whom it is desirable to treat, i.e., a subject at risk of developing age-related cognitive decline, subject suffering from a reduction in cognitive function, and/or subject in need of reduction in heart rate. Subjects in need thereof can also include subjects presenting with the effects of mild cognitive impairment, dementia, Alzheimer's disease, and unexplained memory loss.

In the embodiments herein, the dosage forms can be administered by various routes, including, by way of example and not limitation, parenteral, subcutaneous, intravenous (bolus or infusion), intramuscular, or intraperitoneal routes. Dosage forms for these modes of administration can include conventional forms, either as liquid solutions or suspensions, solid forms suitable for solution or suspension in liquid prior to injection, or as emulsions. In some embodiments, the administration can occur by the placement of the dosage form in a good substance.

In some embodiments, administration of DHA according to the methods described herein can achieve a pharmacokinetic profile of DHA similar to that of a composition comprising DHA and EPA, e.g., Lovaza® (Reliant Pharmaceuticals), even though DHA of the present invention is substantially free of EPA. For example, absorption, incorporation into membranes, hydrolysis by esterases, absorption in the enterocytes, introduction into chylomicrons, very low density lipoproteins (VLDL), low density lipoproteins (LDL), and high density lipoproteins (HDL) of the DHAs can be similar to that observed with a composition comprising DHA and EPA.

Retroconversion is an enzymatic process during which long-chain fatty acids are converted to their related shorter-chain precursor fatty acids though the incremental removal of two-carbon units from the molecule. DHA can be retroconverted to EPA and DPAn-3. See, e.g., Brossard et al., Am. J Clin. Nutr. 64:577-86 (1996). In some embodiments, the DHA of the present invention is retroconverted to a lesser degree (or at a reduced rate) relative to DHA free acid and/or a salt form, or a DHA triglyceride form. For example, in some embodiments, less EPA and/or DPAn-3 is produced in the method using DHAs of the present invention, relative to a method using a DHA free acid and/or salt form, or a DHA triglyceride form.

In the embodiments, herein, administration of the DHA dosage forms can be carried out using various regimens to achieve the desired administered dose. In some embodiments, the dosage form, such as a unit dose, for example a gelatin capsule, is administered once per day, twice per day, three times per day, four times per day, five times per day, six times per day, seven times per day, eight times per day, nine times per day, ten times per day, eleven times per day, or twelve times per day. For instance, for achieving an administered dose of about 0.86 g per day, a single unit dose of about 1 g, where the amount of DHA in the unit dose is abut 86% w/w can be administered once per day. Alternatively, a single unit dose containing about 430 mg of DHA can be administered twice per day, or alternatively two of the same unit doses administered once per day to achieve the same administered dose. In some embodiments, the DHA is administered 3 times a day, e.g., with each meal. In some embodiments, administration of the DHA dosage forms is daily on consecutive days, or alternatively, the dosage form is administered every other day (bi-daily).

The length of treatment can vary depending, by way of example and not limitation, the age of the subject, severity of the disorder, and whether the treatment is for a diagnosed disorder or for prophylactic effect. Accordingly, in some embodiments, administration of the DHA dosage form occurs for at least 28 days, at least 3 months, at least 6 months, or at least 9 months. The method also can include administration of the DHA for shorter periods of time, e.g., once daily for at least 7, 14, 21, or 28 consecutive days. In some embodiments, the length of treatment with DHA can be longer, for example, administering the DHA at the specified dose per day for at least one year; from one to five years, form one to 10 years, from one to twenty years, from five to ten years, or from five to twenty years. In some embodiments, the treatment is maintained continuously as recommended by a prescribing physician. In some embodiments, treatment with the DHA occurs until cognitive function has returned to a preselected target level, the target level being determined by a medical professional. In some embodiments, administration of the DHA dosage form continues even after the cognitive function has reached normal or borderline levels, or to a preselected target level. In some embodiments, the administration of the DHA is administered as a prophylactic measure, before the cognitive function is reduced.

In some embodiments, the invention is directed to a method of increasing cognitive function in a subject, the method comprising administering daily to the subject a dosage form comprising about 200 mg to about 4 g of DHA substantially free of EPA, wherein the dosage form is administered daily for 28 to 365 consecutive days, or for 56 to 185 consecutive days.

In some embodiments, the DHA is administered continuously. The term "continuous" or "consecutive," as used herein in reference to "administration," means that the frequency of administration is at least once daily. Note, however, that the frequency of administration can be greater than once daily and still be "continuous" or "consecutive," e.g., twice or even three times daily, as long as the dosage levels as specified herein are achieved.

In some embodiments, administration of DHA dosage forms can be combined with other regimens (i.e., non-DHA regimens) used to treat reduction in cognitive function. For example, the method of the present invention can be combined with diet regimens (e.g., high omega-3 PUFA diets, etc.), exercise regimens, weight loss regimens, or smoking cessation regimens to improve cognitive function. The methods of the present invention can also be used in combination with other pharmaceutical products to improve cognitive function in a subject (e.g., approved Alzheimer's disease medications).

In some embodiments, administration of DHA dosage form can be combined with other regimens (i.e., non-DHA regimens) used to treat hypertension, high cholesterol levels, heart disease or combination thereof. For example, the method of the present invention can be combined therapeutic with another cardiovascular agent including, for example, an anti-arrhythmic agent, an antihypertensive agent, a calcium channel blocker, a cardioplegic solution, a cardiotonic agent, a fibrinolytic agent, a vasoconstrictor agent, a vasodilator agent, a nitric oxide donor, a potassium channel blocker, a sodium channel blocker, statins; fibrates, ace inhibitors, or a naturiuretic agent.

Statins include atorvastatin, mevastatin, lovastatin, simvastatin, pravastatin and fluvastatin. Examples of other useful fibrates are bezafibrate, ciprofibrate, clinofibrate, clofibrate, etofylline, clofibrate, fenofibrate, gemfibrozil, pirifibrate, simfibrate and tocofibrate; particularly useful are gemfibrozil, fenofibrate, bezafibrate, clofibrate, ciprofibrate and active metabolites and analogues thereof including any relevant fibric acid such as fenofibric acid.

Anti-arrhythmia agents are often organized into four main groups according to their mechanism of action: type I, sodium channel blockade; type II, beta-adrenergic blockade; type III, repolarization prolongation; and type IV, calcium channel blockade, and can include lidocaine, moricizine, mexiletine, tocamide, procainamide, encamide, flecanide, tocamide, phenyloin, propafenone, quinidine, disopyramide, flecamide, propranolol, esmolol, amiodarone, artilide, bretylium, clofilium, isobutilide, sotalol, azimilide, dofetilide, dronedarone, ersentilide, ibutilide, tedisamil, trecetilide, verapamil, diltaizem, digitalis, adenosine, nickel chloride, and magnesium ions.

Examples of cardiovascular agents include vasodilators, for example, hydralazine; angiotensin converting enzyme inhibitors; anti-anginal agents; anti-arrhythmic agents; cardioglycosides, for example, digoxin and digitoxin; calcium antagonists, for example, verapamil and nifedipine; and diuretics.

Other exemplary cardiovascular agents include, by way of example and not limitation, a cyclooxygenase inhibitor such as aspirin or indomethacin, a platelet aggregation inhibitor such as clopidogrel, ticlopidene or aspirin, fibrinogen antagonists or a diuretic such as chlorothiazide, hydrochlorothiazide, flumethiazide, hydroflumethiazide, bendroflumethiazide, methylchlorthiazide, trichloromethiazide, polythiazide or benzthiazide, angiotensin converting enzyme inhibitors such as captopril, zofenopril, fosinopril, enalapril, ceranopril, cilazopril, delapril, pentopril, quinapril, ramipril, lisinopril, and salts of such compounds, angiotensin II antagonists such as losartan, irbesartan or valsartan, thrombolytic agents such as tissue plasminogen activator (tPA), recombinant tPA, streptokinase, urokinase, prourokinase, and anisoylated plasminogen streptokinase activator complex (APSAC, Eminase, Beecham Laboratories), calcium channel blocking agents such as verapamil, nifedipine or diltiazem, thromboxane receptor antagonists such as ifetroban, prostacyclin mimetics, or phosphodiesterase inhibitors.

The non-DHA dosage form can be administered in the same dosage form or simultaneously with the DHA but in a separate dosage form. In some embodiments, the additional non-DHA dosage form can be administered at a time different from the dosage form comprising DHA, e.g., the additional non-DHA dosage form can be administered in the morning and the dosage form comprising DHA can be administered in the evening.

In some embodiments, the DHA of the present invention are administered before the non-DHA regimens. For example, the DHA dosage forms can be first used to increase cognitive function, followed by administration of the non-DHA regimens to maintain (or further lower) the preselected cognitive function level. Alternatively, in some embodiments, the non-DHA regimens are administered first to increase the cognitive function in a subject to a preselected target level, and then the DHA dosage forms of the present invention are administered to maintain (or further lower) the cognitive function levels in the subject. Thus, in some embodiments, the present invention is directed to a method of maintaining cognitive function using the DHA dosage forms described herein, the method comprising (1) administering a non-DHA regimen to a subject to increase cognitive function in the subject, until the cognitive function has reached a preselected level, and (2) administering the DHA dosage forms of the present invention to maintain the preselected cognitive function level.

In some embodiments, the method of the present invention comprises administering daily to a subject a dosage form comprising DHA substantially free of EPA, in conjunction with other triglyceride-lowering methods known to those in the art. For example, the method of the present invention can comprise administering daily to the subject a dosage form comprising DHA substantially free of EPA with one or more additional triglyceride-lowering agents, such as, but not limited to, bile acid binding resins, e.g., cholestyramine and cholestipol; niacin; fibric acid derivatives, e.g., gemfibozil and clofibrate; statins, e.g., lovastatin, pravastatin, atorvastatin and simvastatin; and combinations thereof. The additional triglyceride-lowering agent can be administered in the same dosage form or simultaneously with the DHA but in a separate dosage form. In some embodiments, the additional triglyceride-lowering agent can be administered at a time different from the dosage form comprising DHA, e.g., the additional triglyceride-lowering agent can be administered in the morning and the dosage form comprising DHA can be administered in the evening. In some embodiments, the method of the present invention can comprise administering daily to the subject a dosage form comprising DHA substantially free of EPA in conjunction with a change in diet, e.g., a low total fat diet, a low saturated fat diet, a low dietary cholesterol diet, and/or an increased starch and fiber diet. In some embodiments, the method of the present invention can comprise administering daily to the subject a dosage form comprising DHA substantially free of EPA in conjunction with a change in exercise regimen, e.g., addition of at least 20 minutes of aerobic exercise at least three to five times per week. In some embodiments, the method of the present invention can comprise administering daily to the subject a dosage form comprising DHA substantially free of EPA in conjunction with a weight loss regimen.

The present invention is directed to kits or packages containing one or more dosage forms to be administered according to the methods described herein. A kit or package can contain one dosage form, or more than one dosage forms (i.e., multiple dosage forms). If multiple dosage forms are present in the kit or package, the multiple dosage forms can be optionally arranged for sequential administration. The kits can contain dosage forms of a sufficient number to provide convenient administration to a subject who has a chronic condition and requires long-term administration of the DHA of the present invention. Each dosage form can contain about 100 mg to about 4 g DHA, about 500 mg to about 3 g DHA, or about 1 g to about 2 g DHA and can be intended for ingestion on successive days. For example, in some embodiments, the kit provides dosage forms of a sufficient number for 1, 2, 3 or 4 months of daily administration of the DHA. In some embodiments of the present invention, the kit comprises dosage forms for shorter periods of admmistration, e.g., the kit can contain about 28 to 31 days or more dosage forms for oral administration, each dosage form containing about 500 mg to about 4 g DHA and intended for ingestion on successive days.

The kits of the present invention can optionally contain instructions associated with the dosage forms of the kits. Such instructions can be in a form prescribed by a governmental agency regulating the manufacture, use or sale of pharmaceutical products, which notice reflects approval by the agency of the manufacture, use or sale for human administration to treat a condition or disorder. The instructions can be in any form which conveys information on the use of the dosage forms in the kit according to the methods of the invention. For example, the instructions can be in the form of printed matter, or in the form of a pre-recorded media device.

In the course of examination of a patient, a medical professional may determine that administration of one of the methods of the present invention is appropriate for the patient, or the physician may determine that the patient's condition (e.g., the patient may be suffering dementia or Alzheimer's) can be improved by the administration of one of the methods of the present invention. Prior to prescribing any DHA regimen, the physician can counsel the patient, for example, on the various risks and benefits associated with the regimen. The patient can be provided full disclosure of all the known and suspected risks associated with the regimen. Such counseling can be provided verbally, as well as in written form. In some embodiments, the physician can provide the patient with literature materials on the regimen, such as product information, educational materials, and the like.

The term "consumer information" can include, but is not limited to, an English language text, non-English language text, visual image, chart, telephone recording, website, and access to a live customer service representative. In some embodiments, consumer information will provide directions for use of the DHA dosage forms according to the methods of the present invention, appropriate age use, indication, contraindications, appropriate dosing, warnings, telephone number of website address. In some embodiments, the method further comprises providing professional information to relevant persons in a position to answer consumer questions regarding use of the disclosed regimens according to the methods of the present invention. The term "professional information" includes, but is not limited to, information concerning the regimen when administered according to the methods of the present invention that is designed to enable a medical professional to answer customer questions.

A "medical professional," includes, for example, a physician, physician assistant, nurse practitioner, pharmacist and customer service representative. All of the various aspects, embodiments and options described herein can be combined in any and all variations.

The following examples are illustrative, but not limiting, of the compositions and methods of the present invention. Other suitable modifications and adaptations of the variety of conditions and parameters normally encountered and obvious to those skilled in the art are within the spirit and scope of the invention. Thus, the breadth and scope of the present invention should not be limited by any of the above-described exemplary embodiments, but should be defined only in accordance with the following claims and their equivalents.

### EXAMPLE 1

The effects of DHA (about 900 mg/day) versus placebo was tested to determine whether cognitive measures of memory, attention, and executive function were improved, utilizing the Cambridge Neuropsychological Test Automated Battery (CANTAB) cognitive battery, in healthy elderly subjects with a mild memory complaint. One objective of the study was to determine the effect of DHA administration versus placebo on improving cognitive functions (i.e., working memory, memory retention, attention, processing speed, and executive function) in healthy elderly with a subjective memory complaint. Another objective of the study included determining the effects of DHA on visual acuity, levels of plasma phospholipids, and evaluating the safety and tolerability of the DHA dose administered.

The study was a randomized, double-blind, placebo-controlled, parallel, multi-center design study to evaluate the effects of an oral dose of DHA on cognitive functions in healthy elderly with a subjective memory complaint. The study consisted of a screening/baseline period, and a 24 week treatment period

Eligible male or female subjects were randomized to one of 2 treatment groups, a DHA group and a placebo group.

The DHA group was provided 800 mg soft-gelatin capsules, each containing about 300 mg DHA as a triglyceride from algal oil (DHASCO®-S oil, 40% DHA). In addition to 40% DHA, DHASCO®-S oil was formulated to contain 40-60% saturated, mono- and polyunsaturated fatty acids, 400 ppm ascorbyl palmitate, 2000 ppm mixed tocopherols, 2000 ppm rosemary extract, 3% orange-flavoring, and 1% natural masking agent. Subjects were instructed to take three 800 mg capsules/day, i.e., about 900 mg DHA/day.

The placebo group was provided 800 mg soft-gelatin capsules containing 48% com oil, 48% soy oil, 3% orange-flavoring and 1% natural masking agent. The capsules were formulated to also contain 400 ppm ascorbyl palmitate, 2000 ppm mixed tocopherols, and 2000 ppm rosemary extract. Similar to the DHA group, subjects were instructed to take three 800 mg capsules/day.

Each group was provided its capsules in a bottle. Each bottle contained one month's supply of capsules. Subjects were instructed to take the capsules with food at the same time each day, and on consecutive days, i.e., daily, throughout the study period (24 weeks), starting the day of the baseline visit after all assessments are completed. Subjects were instructed to not alter their normal diet during the study. A DHA food frequency questionnaire was administered throughout the study to assess dietary intake of omega-3 fatty acids and compliance with the dosage regimen.

The subjects were evaluated at baseline, at 1 month, 3 months and at 6 months ("end of treatment efficacy assessments"). Subjects having a subjective memory complaint and who had a Logical Memory (WMS III) test screening raw score one standard deviation or greater below the mean of a younger population were recruited. In some studies, this is known as age-related cognitive decline, and also known as age-associated memory impairment (AAMI), or age consistent memory decline. Enrollment obtained about 485 subjects total.

During the Screening Period, subjects were selected on the basis of specific inclusion and exclusion criteria, including a screening Logical Memory sub-test of the Wechsler Memory Scale (WMS III). See e.g., Wechsler, D., Administration and Scorning Manual, 3rd ed. San Antonio, TX: Harcourt Brace & Company, 1997. In addition to the Logical Memory sub-test, the Mini-Mental State Examination (MMSE), Geriatric Depression scale, and a test of visual acuity and a DHA Food Frequency Questionnaire were administered to all subjects at screening. See e.g., Folstein et al., Psychiatr. Res. 12:189-198 (1975); Yesavage J., et al., J Psychiatr. Res. 1:37-49 (1982). Eligible subjects who meet the screening criteria were given a pre-baseline training session with the computerized cognitive battery, CANTAB.

Pre-baseline testing with the CANTAB occurred to serve as a training session and to familiarize the subject with the computer system. Morris R, et al., Computer Aided Assessment of Dementia: Comparative Studies of Neuropsychological Deficits in Alzheimer Type Dementia, and Parkinson's Disease (1987). Seven (+/-2) days following this pre-baseline testing, baseline measurements were taken, including baseline laboratory measures, vital signs, and the CANTAB cognitive battery. At the baseline session, subjects were stratified based on age (55-69; ≥70) and randomly assigned to receive about 900 mg DHA, or placebo. Capsules, either DHA or placebo, as randomly assigned, was given to each subject at the end of the baseline visit. Subjects were instructed to take 3 capsules everyday for the ensuing 24 weeks and were supplied initially with 3 months' worth of capsules. At the end of 4 weeks, subjects returned to the site for compliance and safety assessments. Subjects returned to the site at 12 weeks for safety and efficacy assessments and were re-supplied with the final 3 months' worth of capsules. Subjects returned to the site at 24 weeks for the "end of study" efficacy and safety assessments. Compliance was assessed by food frequency questionnaires, returned capsule counts, and by plasma DHA levels (taken at 24 weeks). The CANTAB cognitive battery consists of tests of memory, learning, attention, problem-solving, and executive function (i.e., planning and organizational skills). See e.g., Fowler et al., Applied Neuropsychology 2:72-78 (1995); Robbins et al., Dementia 5:266-281 (1994); and De Luca et al., J Clinical and Experimental Neuropsychology 25:242-254 (2003).

The computerized battery operated using a touchtone screen and responses were recorded and stored in a computer database. Data was transferred to a central database formatted for variables of interest and analysis. Baseline, week 12 and week 24 CANTAB assessments were collected for each subject for analysis. The CANTAB instructions and set up were administered by examiners who were blinded to the subject's treatment.

Subjects eligible for the study met the inclusion criteria listed below.
- Be male or female, and at least 55 years old
- Had a subjective memory complaint and a Logical Memory subtest (of the WMS III) raw score one standard deviation or greater below the mean of a younger population (≤28 immediate recall cut-off total score; or ≤15 delayed recall cutoff total score).
- Had the ability to understand the requirements of the study and were willing to provide written informed consent (as evidenced by signature on an informed consent document approved by an Institutional Review Board [IRE]) and agree to abide by the study restrictions and return for the required assessments.
- If taking non-prohibited medication, were on a stable drug regimen (in prior 3 months)

To be eligible for entry into the study, the subject must not have met any of the exclusion criteria listed below.
- Had a screening MMSE<26
- Consumed greater than 200 mg/day DHA as assessed on the DHA Food Frequency Questionnaire in the prior 2 months to screening
- Used nutritional fish oil, flaxseed oil, omega-3 supplements, or huperzine in the prior 2 months to screening.
- Used acetylcholinesterase inhibitors or memantine, in the prior 2 months to screening.
- Used major anti-psychotics or major anti-depressants.
- Used lipase inhibitors such as Xenical® (orlistat).
- History of major medical conditions including ischemic stroke, head trauma with loss of consciousness, epilepsy, psychosis, vascular dementia, depression (Geriatric Depression[15-item] >5), myocardial infarction (within 1 year), uncontrolled diabetes, or blindness.
- History of major surgery within the past 6 months.
- Used or history of drug and/or alcohol abuse within 5 year.
- Administration of any investigational product within the past 30 days.
- Inability to swallow capsules.

Blood samples of the subjects were collected at baseline and at the week 24 or "end of study" visit to determine phospholipid levels. Samples were collected into 10 ml vacutainer EDTA tubes, centrifuged at 3000 rpm and plasma was stored at -80°C until analyzed. Plasma phospholipid fatty acids were analyzed as described previously. See, e.g., Arterburn, L.M., et al., Lipids 42:1011-1024 (2007). The fatty acid profiles were expressed as a percent of the total fig of fatty acid (weight percent).

A primary efficacy endpoint of the CANTAB test score on the Paired Associate Learning (PAL) test at 24 weeks of treatment was used as the primary efficacy outcome. The primary analysis was adjusted for baseline scores, education, site, and the stratification factor of age. Secondary analyses were based on the model above with the addition of the factor, concomitant statin medication use. The PAL subtest of the CANTAB is a test of visuospatial cued recall memory that detects changes in episodic memory. See e.g., de Jager et al., Psychological Medicine 32:483-491(2002). Tests of episodic memory such as the PAL have been shown in longitudinal studies and studies of MCI patients to be highly predictive of the early stages of Alzheimer's disease and may be very effective at identifying at-risk individuals. See, e.g., Nestor et al., Nat. Med. 10:S34-41 (2004); and Backman et al., Neuropsychology 19:520-531 (2005).

The secondary efficacy endpoints included:
- CANTAB tests of executive function and planning: Spatial Working Memory (SPM) and Stockings of Cambridge (SOC)
- CANTAB Verbal Recognition Memory test (VRM)
- CANTAB Pattern Recognition test (PRM)
- Visual acuity (measured by Snellen Eye chart; corrected vision)
- Memory Functioning Questionnaire: Frequency of Forgetting-10 (Self-rating scale)
- Activities of Daily Living (ADCS-ADL PI) scale (Self-rating)
- Clinical measures of cognitive function and computerized assessments were administered by (CANTAB) certified examiners at each site blinded to the subject's treatment.
- Description of CANTAB Battery of tests

The 15 tests in the CANTAB battery are divided into five main types of task:
- training and screening
- attention and memory
- non-strategic learning and memory
- sustained attention
- frontal/executive tasks

See, e.g., www site at cantab.com (accessed April 14, 2009) and cognitive tests provided by Cambridge Cognition Ltd. (Cambridge, England).

The training and screening tests included a motor screening, Big/Little Circle assay, and Reaction Time assay. The motor screening test is common to all of the CANTAB batteries, and was given at the beginning of a test session. A series of crosses is shown in different locations on the screen. After a demonstration of the correct way to point using the forefinger of the dominant hand, the subjects must point to the crosses in turn. This motor screening test has two purposes: (i) to act as a training procedure to ensure that the subjects can point accurately, and (ii) to provide measures of both speed and accuracy that provide an index of the subjects' motor skill. The Big/Little Circle (BLC) assay comprises a series of pairs of circles, one large and one small, are presented to the subject The subject is instructed first to point to the smaller of the two circles, and then, after 20 trials to point to the larger circle. This visual discrimination test is designed to train a subject to: (i) follow an explicit instructional rule, and (ii) reverse a rule. The Reaction Time assay (RTI) has three purposes: (i) to train the subject in the skills related to holding down the press pad and touching the screen, (ii) to provide a screen for the ability to acquire and perform this motor skill, and (iii) to act as a simple single and multiple choice reaction time task. The subject must touch the screen when a yellow dot is displayed. For the multiple choice reaction time test, the dot may be shown in one of five locations.

The Attention and memory tests included (i) a matching to sample visual search, (ii) a delayed matching to sample assay, (iii) a pattern recognition memory assay, (iv) a spatial recognition memory assay, and (v) spatial span assay. The Matching to Sample Visual Search (MTS) is a speed/accuracy trade-off task, testing the subject's ability to match visual samples and measuring their reaction and movement time. An abstract pattern composed of four colored elements is presented in the middle of the screen. After a brief delay, a varying number of similar patterns is shown in a circle of boxes around the edge of the screen. Only one of these matches the pattern in the centre of the screen and the subject must indicate which it is by touching it. The number of patterns in the circle may be 1,2,4 or 8, and the incorrect patterns are composed of juggled elements of the sample pattern or juggled distracter elements. This test is not contained in CANTABexpedio. The Delayed Matching to Sample (DMS) is a test of perceptual matching, immediate and delayed visual memory, in a four-choice simultaneous and delayed recognition memory paradigm. The subjects are shown a complex visual pattern (the sample) and then, after a brief delay, four patterns. In some trials, the sample and the choice patterns are shown simultaneously, whereas in others a delay (of 0, 4 or 12 seconds) is introduced between covering the sample pattern and showing the choice patterns. The Pattern Recognition Memory (PRM) is a test of visual pattern recognition memory in a 2-choice forced discrimination paradigm. A sequence of visual patterns is presented in the centre of the screen. These patterns are designed so that they cannot easily be given verbal labels. In the recognition phase, the subjects are required to choose between a pattern they have already seen and a novel pattern. The Spatial Recognition Memory (SRM) is a test of spatial recognition memory in a 2-choice forced discrimination paradigm. A white square is displayed in sequence in five different places on the screen. In the recognition phase, the subjects see a series of five pairs of squares, one of which is in a place previously seen in the presentation phase, and one of which is a distracter. The five squares are shown in reverse order. The Spatial Span (SSP) test gives a measure of the subject's spatial memory span. A set of white squares is shown on the screen. Some of the squares change in color, one by one, in a variable sequence. At the end of each sequence a tone indicates that the subject should touch each of the boxes colored by the computer in the same order as they were originally presented. The number of squares ranges from 2 to 9 squares.

The Non-strategic learning and memory tests include a paired associates learning test. The Paired Associates Learning (PAL) test is a form of delayed response procedure, which tests two different aspects of the ability to form visuo-spatial associations: (i) the number of patterns placed correctly on the first presentation of each trial gives an index of 'list memory', (ii) the number of repeat, reminder presentations needed for the subject to learn all the associations provides a measure of 'list learning.' Six boxes are displayed on the screen. All are opened in a randomized order. One or more of them will contain a pattern. The subject is required to remember patterns associated with different locations on the screen, and during the test phase, as each pattern is presented, point to the appropriate location. The test starts at a very simple level and gradually increases in difficulty.

The Sustained attention test includes the Rapid Visual Information Processing test. The Rapid Visual Information Processing (RVP) test of visual sustained attention with a small working memory component. A white box is displayed in the centre of the computer screen, inside which digits, from 2 to 9, are displayed in a pseudo-random order, at the rate of 100 digits per minute. The subject must detect consecutive odd or even sequences of digits (for example, 2-4-6) and respond by pressing the touch pad.

The Fronta/executive tasks include the (i) Spatial Working Memory assay, (ii) the ID/ED shift assay, and (iii) the Stockings of Cambridge assay. The Spatial Working Memory (SWM) is a test of the subject's ability to retain spatial information and to manipulate remembered items in working memory. It is a self-ordered task, which also assesses heuristic strategy. A trial begins with a number of colored squares (boxes) being shown on the screen. The overall aim is that the subject should find a blue 'counter' in each of the boxes and use them to fill up an empty column on the right hand side of the screen. The subject must touch each box in turn until one opens with a blue 'counter' inside (a search). Returning to an empty box already sampled on this search is an error. The ID/ED shift (IED) test examines a subject's ability to attend to the specific attributes of compound stimuli, and to shift that attention when required. Two artificial dimensions are used in the test; color-filled shapes and white lines. Simple stimuli are made up of just one of these dimensions, whereas compound stimuli are made up of both, namely white lines overlying color-filled shapes. Subjects progress through the test by satisfying a set criterion of learning at each stage (6 consecutive correct responses). If at any stage the subject fails to reach this criterion after 50 trials, the test ends. The Stockings of Cambridge (SOC) test is a test of spatial planning based upon the 'Tower of London' test. The subject is shown two displays containing three colored balls. The displays can easily be perceived as stacks of colored balls held in stockings or socks suspended from a beam. This arrangement assists subjects to come to grips with some of the rules of the problems which involve 3-D concepts, and to fit in with the verbal instructions. The subject must use the balls in the lower display to copy the pattern shown in the upper one.

Additional tests are included in CANTABeclipse and are available in the CANTABelect batteries. The Affective Go/No-go (AGN) test assesses information processing biases and inhibitory control for positive and negative stimuli. The test consists of several blocks, each of which presents a series of words taken from two of three different Affective categories: Positive (for example, joyful), Negative (for example, hopeless), and Neutral (for example, element). The subject is given a target category (for example, positive) and is asked to press the press pad when they see a word matching this category, giving latency and total correct/incorrect scores. The Verbal Recognition Memory (VRM) test assesses immediate and delayed memory of verbal information under free recall and forced choice recognition conditions. In this test, the subject is shown 12 words and then asked to: (i) produce as many of the words as possible immediately following the presentation (ii) recognize the words they have seen before from a list of 24 words containing the original 12 words and 12 distractors following a delay of 20 minutes, (iii) recognize the words they have seen before from another list of 24 words containing the original 12 words and 12 new distractors. All of the words included in this test are of high imageability. The distractors are closely matched to the target words on frequency and word length.

Other secondary measures included self-assessment tests of memory (Frequency of Forgetting-10 scale) (Zelinski, E.M. et al., Aging and Mental Health 8:293-306(200)), Alzheimer's Disease Cooperative Study-Activities of Daily Living Prevention Instrument (ADCS-ADL PI scale) (Galasko, D., Alzheimer Dis. Assoc. Disord. 20: S 152-69 (200)), Mini-Mental State Examination (MMSE) (Folstein et al., Pyschiatr. Res. 12:189-198 (1975) and the Geriatric Depression Scale (Yesavage, J., et al., J Psychiatr. Res. 1:3749 (198)).

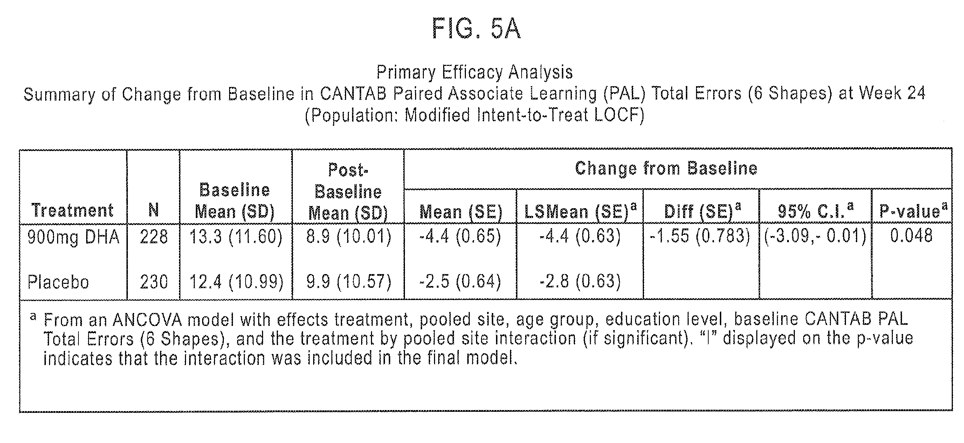

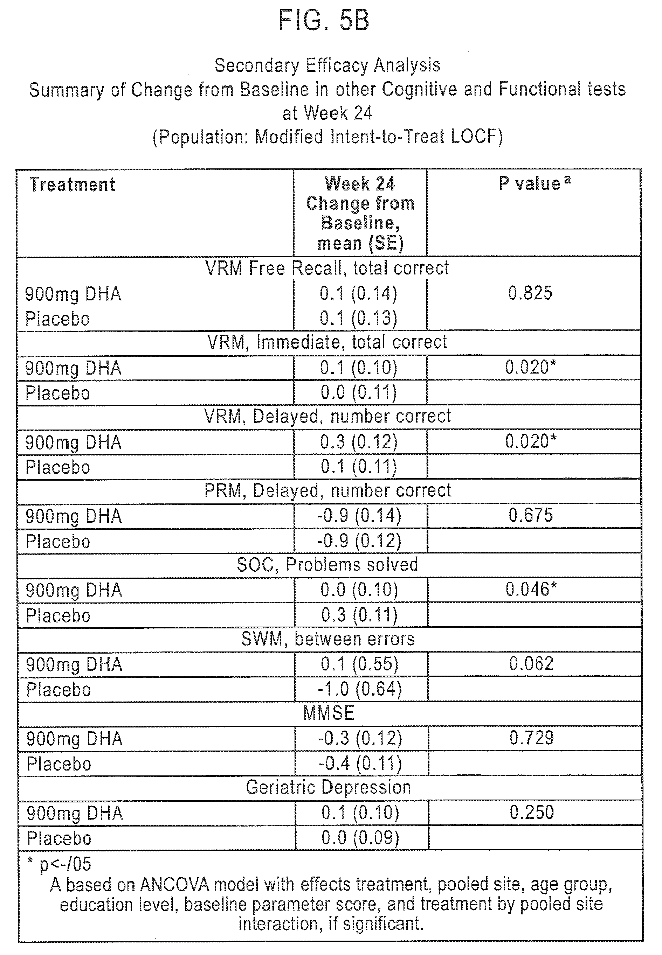

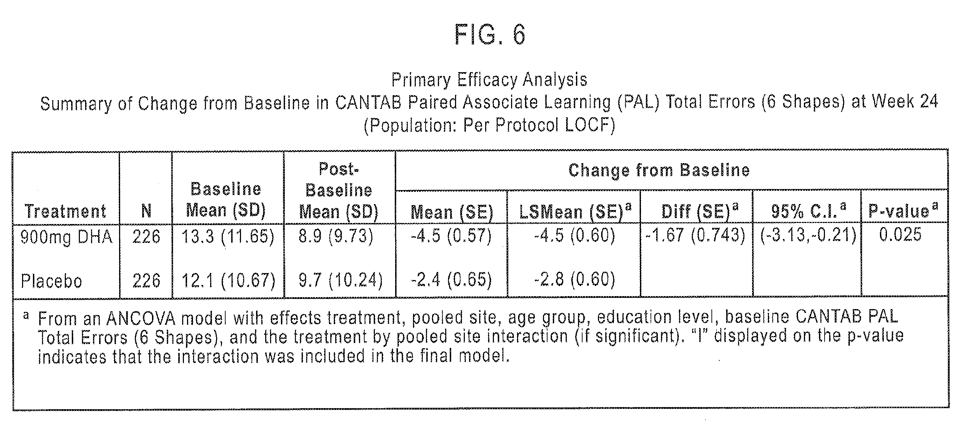

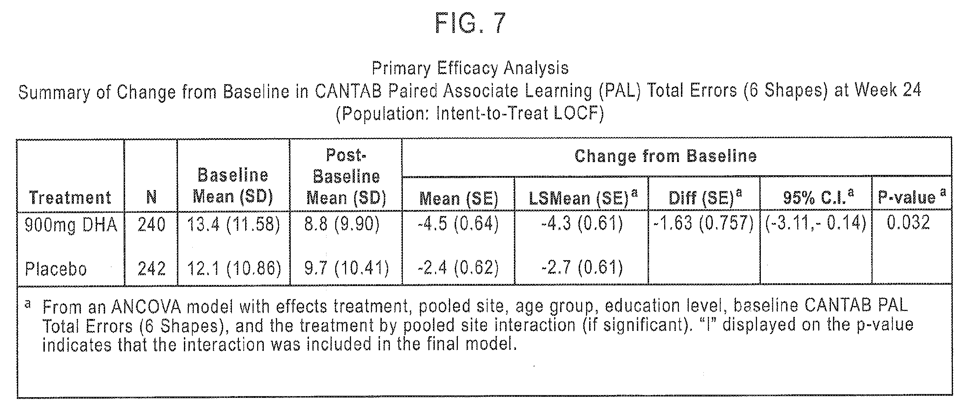

**Results.** A 90% study completion rate was achieved with a mean age of 70±9, and mean education level of 14.6±2.6 years. For Primary Efficacy, an ITT analysis demonstrated significantly fewer errors made on the PAL 6 pattern stage test with about 900 mg/d DHA versus placebo at six months compared to baseline (diff. score -1.63±0.76, p<0.03). See, e.g., FIG. 5A. The delayed Logical Memory test of the WMSIII was highly predictive of the PAL change (p<0.001). CANTAB Verbal Recognition Memory test also showed significant change from baseline correct responses for both immediate (diff. 0.4±0.17, p<0.02) and delayed recognition (diff. 0.4±0.18, p<0.02) with DHA compared to placebo. CANTAB Pattern Recognition and Spatial Working Memory test responses were not significantly different between the 2 groups. See FIGS. 5-7.

A significant decrease in heart rate (DHA change from baseline of -3.2 vs. -1 BPM, p<0.03) was also observed and was highly correlated with week 24 plasma DHA levels (p<0.01). Blood pressure and body weight remained unchanged between groups. Abeta_{1-40,1-42} and hs-CRP plasma levels were not significantly different. Plasma phospholipid DHA levels doubled (3.2 to 6.4 weight%, p<0.001), and were correlated with the PAL response (p<0.04) and decreases in total cholesterol (p<0.01). Compliance was >82%, no treatment-related SAEs were reported and the number of subjects with SAEs was equivalent across the 2 study arms.

Plasma phospholipid DHA levels significantly increased by an average 3.2 weight % in the DHA-treated group by week 24 (mean baseline level of 3.2 weight %). See FIG. 10. The placebo group showed a slight decrease (mean change of -0.08 weight %) in plasma phospholipid DHA over 24 weeks (diff. score of 3.15±0.16, p<0.001). Corresponding decreases in plasma phospholipid arachidonic acid (ARA) (mean change of -1.37 weight %) were seen with DHA supplementation and were significantly different from the ARA change from baseline level (mean change of -0.12 weight %) in the placebo group (p<0.001). Plasma phospholipid EPA levels were slightly increased in the DHA supplemented group (mean change of 0.16 weight %) and slightly decreased in the placebo group (mean change of -0.06 weight %), p<0.001. The plasma phospholipid DPAn-6 levels were also significantly increased with DHA administration over 24 weeks (mean change of 0.37 weight %), while DPAn-6 levels were basically unchanged in the placebo group (mean change of -0.004 weight %), p<0.001. The plasma phospholipid DHA levels at week 24 (measured as log plasma concentration) were significantly correlated with the change from baseline PAL response, r = -1.85, p<0.038.

Compliance was primarily measured by the change from baseline plasma DHA levels at week 24. A change greater than 1.5 weight % (based on historical dose response plasma DHA levels) was considered compliant for the DHA-treated group. Based on this change, > 82% of the DHA-treated subjects were compliant. Compliance was > 99% in the placebo group using ≤ 1.5 weight % change. A secondary measure of compliance, using capsule counts demonstrated > 91% compliance with the clinical material administered across both groups. Compared to baseline, the week 24 DHA FFQ also showed 98% compliance for dietary DHA intake ≤ 200 mg/day for both groups. A compliers analysis of the primary endpoint revealed similar improvement in the DHA group versus placebo (diff. score - 1.3±0.89; -3.1, 0.4 95% CI) but did not reach significance, p<0.13 due to a loss of 85 people in the analysis who terminated the study early, did not have plasma level data, or did not comply with the study dosing regimen.

The results of this study demonstrated that oral DHA (about 900 mg/day) supplementation improves memory and learning in healthy older adults with a mild memory complaint as measured by a significant reduction in the number of visuospatial learning and episodic memory errors over a six month period. Supplementation with DHA resulted in an approximate 2-fold reduction in CANTAB PAL 6 pattern errors compared to the decrease in the number of errors with placebo treatment. The DHA-induced cognitive changes were significantly correlated with log DHA plasma levels at the end of the study. Compared to age-associated normative data on the CANTAB PAL, results on the PAL with DHA supplementation show a 7 year improvement in the PAL test performance versus a 3.6 year improvement with placebo. The mean baseline PAL errors in the DHA group (13.4 ±11.5) correspond to an age of 72.6 years and after 6 months of DHA supplementation, mean PAL errors are 8.8 ±9.9, corresponding to 65.6 years of age on this test. Placebo-treated subjects had a PAL normative age of 70•63 years at baseline and after 6 months displayed a normative age of 66•99, likely due to a small learning effect on the test. The CANTAB PAL test has been well-characterized as an episodic memory test that depends upon mnemonic processes of the medial temporal lobe. See, e.g., Blackwell, A.D., et al., Dement. Geriatr. Cogn. Disord. 17:42-8 (2004); and de Jager, C.A., et al., Psychological Medicine 32: 483-491 (2002). The PAL has been shown to discriminate between healthy controls, mild cognitively impaired individuals, Alzheimer's disease patients and depressed individuals. See, e.g., Egerhazi, A., et al., Prog Neuropsychopharmacol Biol Psychiatry 31:746-51 (2007); and Swainson, R., et al., Dement. Geriatr. Cogn. Disord. 12:265-80 (2001). These studies have demonstrated that changes in the PAL test are very accurate in detecting early memory impairment that is indicative of age-related cognitive decline and may be predictive of preclinical Alzheimer's disease. Changes on this task with DHA indicate that DHA omega-3 fatty acid supplementation can ameliorate early memory and learning deficits associated with aging. The Logical Memory test of the WMS, an episodic memory test, was used to objectively identify subjects with age-related cognitive decline and was highly predictive of the PAL improvement with DHA. Adjusting for the Logical Memory delayed recall score, strengthened DHA-induced changes on PAL, confirming positive effects on episodic memory function. Other cofactors, such as family history of dementia, and concomitant statin use were also predictive of DHA's response on the PAL, suggesting potential genetic and cardiovascular susceptibility factors that may influence DHA's effects on cognition. Age was not a significant predictor of the primary endpoint changes, since subject randomization stratified for age and age was a cofactor in the analyses, although greater PAL errors were seen in the older age group.

CANTAB Verbal recognition memory, another episodic memory task was also modestly improved with DHA supplementation, lending support to the idea that DHA is enhancing early, potentially isolated memory functions in aging adults. Changes in working memory (SWM) and executive function (SOC), cognitive functions that are typically impaired in multi-domain MCI and later stages of Alzheimer's disease, were not found with DHA supplementation in the subjects. The MMSE, a measure of global cognitive function, as well as the Geriatric Depression scale, a measure of depression in older adults, were unchanged over the 6 month study and were not different between groups, indicating a relatively stable, healthy older adult population of subjects with a mild memory deficit was studied. Likewise, ADL and self-perceptive memory assessments exhibited little impairment, were unaffected by DHA supplementation and showed slight but non-significant improvements by both groups over the 6 month timeframe of the study.

The dose of about 900 mg/day algal DHA was well tolerated. Good compliance was demonstrated with this dose and drop outs from the study were only 10% after six months. Plasma phospholipid fatty acid levels were altered, as expected, at this dose with a 3.2 weight% increase in DHA levels and a corresponding 1.4 weight % decrease in ARA levels. ARA levels typically decline when DHA consumption increases. Small changes in EPA levels can be due to minor fatty acid retroconversion from DHA or due to the small quantity of EPA in the oil, while increases in DPAn-6 were expected due also to the presence of this fatty acid in the oil blend. The significance of the DPAn-6 increase may serve a role in neuronal signal transduction and phosphorylation of tau protein, but this remains to be explored further. See, e.g., Green, K.N., et al., J Neurosci. 27:4385-95 (2007).

This study confirms clinically a significant beneficial effect of DHA on improving memory and learning functions in healthy adults with age-related cognitive decline. The positive findings indicate that about 900 mg/d DHA serves as a nutritional neuroprotective agent in improving the pattern of very early cognitive deficits associated with aging. These cognitive changes likely occur as a consequence of the normal aging process or may be detected prior to a diagnosis such as MCI or mild Alzheimer's disease. DHA appears to have a significant impact on early episodic memory changes. Changes in episodic memory, as demonstrated by the PAL test have been shown by others to be predictive of pre-clinical Alzheimer's disease. See, e.g., Fowler, K.S., et al., J Int. Neuropsychol. Soc. 8:58-71 (2002); and Blackwell, A.D., et al., Dement. Geriatr. Cogn. Disord. 17:42-8 (2004). The cognitive benefits of DHA found in our study impact upon a major health concern of memory loss in older adults and provide a well tolerated and effective nutritional supplement that improves brain health with aging.

In summary, a six month supplementation with DHA (about 900 mg/d) improved memory function in healthy older adults with age-related cognitive decline. This improvement on the PAL is associated with a shift in the normative distribution to a younger age group. DHA appears to have a significant impact on early episodic memory changes which may be predictive of pre-clinical AD. A six month supplementation with DHA (about 900 mg/d) also decreases heart rate in healthy older adults with age-related cognitive decline. DHA shows beneficial cardiovascular effects with an excellent safety profile in this older adult population.

All of the various embodiments or options described herein can be combined in any and all variations. While the invention has been particularly shown and described with reference to some embodiments thereof, it will be understood by those skilled in the art that they have been presented by way of example only, and not limitation, and various changes in form and details can be made therein without departing from the spirit and scope of the invention. Thus, the breadth and scope of the present invention should not be limited by any of the above described exemplary embodiments, but should be defined only in accordance with the following claims and their equivalents.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

All publications and patent applications mentioned in this specification are herein incorporated by reference in their entirety to the same extent as if each individual publication or patent application was specifically and individually indicated to be incorporated by reference.

Various preferred features and embodiments of the present invention will now be described with reference to the following numbered paragraphs (paras).
1. A method of treating age related cognitive decline (ARCD) or mild cognitive impairment (MCI), comprising administering to a subject in need thereof about 0.8 g to about 4 g of docosahexaenoic acid (DHA) per day in a dosage form substantially free of eicosapentaenoic acid (EPA) and substantially free of arachidonic acid (ARA).
2. The method of para 1, wherein the EPA is less than 2% (w/w)of the total fatty acid content of the dosage form.
3. The method of para 1, wherein the EPA is less than 0.1% (w/w)of the total fatty acid content of the dosage form.
4. The method of para 1, wherein the EPA is not detectable in the dosage form.
5. The method of para 1, wherein the ARA is less than about 2 % (w/w) of the total fatty acid content of the dosage form.
6. The method of para 5, wherein the ARA is less than about 0.1% (w/w) of the total fatty acid content of the dosage form.
7. The method of para 6, wherein the ARA is not detectable in the dosage form.
8. The method any one of paras 1 to 6, wherein the DHA is derived from an algal source.
9. The method of para 8, wherein the algal source is *Crypthecodinium cohnii, Thraustochytrium,* or *Schizochytrium sp.*
10. The method of any one of paras 1 to 9, wherein about 0.84 g to about 4 g of DHA is administered per day to the subject.
11. The method of para 10, wherein about 0.84 g to about 1.5 g of DHA is administered per day to the subject.
12. The method of para 10, wherein about 0.84 mg to about 1.0 g of DHA is administered per day to the subject.
13. The method of any one of paras 1 to 12, wherein the dosage form is administered once per day, twice per day, three times per day, four times per day, five times per day, six times per day, seven times per day, eight times per day, nine times per day, ten times per day, eleven times per day, or twelve times per day.
14. The method of paras 1 to 13, wherein the DHA is administered in at least one unit dose.
15. The method of para 14, wherein the unit dose comprises about 0.2 g to about 1 g of DHA.
16. The method of para 15, wherein the unit dose has a total weight of about 0.20, 0.35, 0.40, 0.45, 0.50, 0.55, 0.60, 0.65, 0.70, 0.75, 0.80, 0.85, 0.90, 0.95, 1.0 g or 1.05 g.
17. The method of para 16, wherein the DHA in the unit dose is about 30% to about 99.5% (w/w) of the total fatty acid content of the unit dose.
18. The method of para 16, wherein the DHA in the unit dose is about 35% to about 65% (w/w) of the total fatty acid content of the unit dose.
19. The method of any one of paras 14 to 18, wherein the unit dose is characterized by one or more of the following amount of fatty acids:
   (a) capric acid is less than 1% (w/w) ;
   (b) lauric acid is less than 1% (w/w) ;
   (c) myristic acid is less than 1% (w/w) ;
   (d) palmitic acid is less than 1% (w/w)
   (e) palmitoleic acid is less than 1% (w/w) ;
   (f) stearic acid is less than 1% (w/w) ;
   (g) oleic acid is less than 1% (w/w) ;
   (h) linolec acid is less than 1% (w/w) ;
   (i) α-linolenic acid is less than 1% (w/w) ;
   (j) docosapentaenoic acid 22:5n-3, 22:5w3 (DPAn3) is less than 1% (w/w) ;
   (k) docosapentaenoic acid 22:5n-6, 22:5w6 (DPAn6) is less than 1% (w/w); and
   (l) 4,7,10,13,16,19,22,25 octacosaoctaenoic acid (C28:8) is less than 1% (w/w);
      of the total fatty acid content of the unit dose.
20. The method of para 14, wherein the DHA in the unit dose comprises about 40% to about 50% (w/w) of the total weight of the unit dose.
21. The method of para 20, wherein the DHA in the unit dose comprises about 40% to about 45% (w/w) of the total fatty acid content of the unit dose.
22. The method of para 20 or 21, wherein the unit dose is characterized by one or more of the following amount of fatty acids:
   (a) capric acid (C10:0) is about 2% or less;
   (b) lauric acid (C12:0) is about 6% or less;
   (c) myristic acid (C14:0) is about 20% or less;
   (d) palmitic acid (C16:0) is about 20% or less;
   (e) palmitoleic acid (C16:1n-7) is about 3% or less;
   (f) stearic acid (C18:0) is about 2% or less;
   (g) oleic acid (C18: 1n-9) is about 40% or less;
   (h) linoleic acid (C18:2) is about 5% or less;
   (i) nervonic acid (C24:1) is about 2% or less: and
   (j) Others is about 3% or less;
      of the total fatty acid composition of the unit dose.
23. The method of para 14, wherein the DHA in the unit dose comprises about 35% to about 45% (w/w) of the total weight of the unit dose.
24. The method of para 23, wherein the DHA in the unit dose comprises about 35% to about 45% (w/w) of the total fatty acid content of the unit dose.
25. The method of para 23 or 24, wherein the unit dose is characterized by one or more of the following amount of fatty acids:
   (a) myristic acid (C14:0) is about 12% or less;
   (b) palmitic acid (C16:0) is about 28% or less;
   (c) stearic acid (C18:0) is about 2% or less;
   (d) oleic acid (C18:1n-9) is about 8% or less;
   (e) linoleic acid (C18:2) is about 2% or less;
   (f) arachidonic acid (C20:4) is about 2% or less;
   (g) eicosapentaenoic acid (C20:5) is about 3% or less;
   (h) docosapentaenoic acid (22:5n-6) is about 18% or less; and
   (i) Others is about 10% or less;
      of the total fatty acid composition of the unit dose.
26. The method of para 14, wherein the DHA in the unit dose comprises about 55% to about 57%% (w/w) of the total weight of the unit dose.
27. The method of para 26, wherein the DHA in the unit dose comprises about 55% to about 67% (w/w) of the total fatty acid content of the unit dose.
28. The method of para 26 or 27, wherein the unit dose is characterized by one or more of the following amount of fatty acids:
   (a) capric acid (C10:0) is 2% or less;
   (b) lauric acid (C12:0) is about 6% or less;
   (c) myristic acid (C14:0) is about 20% or less;
   (d) palmitic acid (C16:0) is about 15% or less;
   (e) palmitoleic acid (C16:1n-7) is about 5% or less;
   (f) stearic acid (C18:0) is about 2% or less;
   (g) oleic acid (C18:1n-9) is about 20% or less;
   (h) linoleic acid (C 18:2) is about 2% or less;
   (i) nervonic acid (C24:1) is about 2% or less; and
   (l) Others is about 3% or less;
      of the total fatty acid composition of the unit dose.
29. The method of any one of paras 19 to 28, wherein the DHA comprises an algal oil.
30. The method of any one of paras 19 to 28, wherein the DHA comprises a triglyceride.
31. The method of para 14, wherein the DHA in the unit dose comprises a ratio of wt% of DHA to wt% DPAn6 of about 2.5 to about 2.7.
32. The method of para 31, wherein the DHA in the unit dose comprises greater than about 67% to about 72% (w/w) of the total fatty acid content of the unit dose.
33. The method of para 32, wherein the unit dose comprises DPAn6 of about 15% to about 30% (w/w) of the total weight of the fatty acid content of the unit dose.
34. The method of para 15, wherein the DHA in the unit dose comprises about 85% to about 96% (w/w) of the total weight of the unit dose.
35. The method of para 34, wherein the DHA in the unit dose comprises about 85% to about 99.5% (w/w) of the total fatty acid content of the unit dose.
36. The method of para 34 or 35, wherein the unit dose is characterized by one or more of the following amounts of fatty acid:
   (a) capric acid (C10:0) is less than 0.1% or not detectable;
   (b) lauric acid (C12:0) is less than 0.1% or not detectable;
   (c) myristic acid (C14:0) is about 0.1% or less or not detectable;
   (d) palmitic acid (C16:0) is about 0.5% or less;
   (e) palmitoleic acid (C16:1n-7) is about 0.5% or less;
   (f) stearic acid (C18:0) is about 0.5% or less or not detectable;
   (g) oleic acid (C18:1n-9) is about 4% or less;
   (h) linoleic acid (C18:2) is less that 0.1% or not detectable;
   (i) arachidonic acid (C20:4) is about 0.1% or less;
   (i) eicosapentaenoic acid (C20:5) is less that 0.1 % or not detectable;
   (j) docosapentaenoic acid (22:5n-6) is about 3% or less; and
   (j) Others is about 1% or less;
      of the total fatty acid composition of the unit dose.
37. The method of any one of paras 34 to 36, wherein the DHA comprises a DHA ester.
38. The method of para 37, wherein the DHA ester is an alkyl ester.
39. The method of para 38, wherein the DHA alkyl ester is a DHA methyl ester, ethyl ester, propyl ester, or combinations thereof.
40. The method of para 39, wherein the unit dose comprises about 430 mg to about 480 mg of DHA ethyl ester.
41. The method of para 39, wherein the unit dose comprises about 860 mg to about 950 mg of the DHA ethyl ester.
42. The method of para 41, wherein the unit dose comprises about 870 mg to about 930 mg of the DHA ethyl ester.
43. The method of any one of paras 1 to 42, wherein the dosage form further comprises one or more tocopherols, one or more tocotrienols, or a combination thereof.
44. The method of para 1, wherein the dosage form is administered once daily.
45. The method of para 1, wherein the dosage form is administered daily for 1 to 10 years.
46. The method of para 1, wherein the dosage form is administered daily for 1 to 12 consecutive months.
47. The method of para 1, wherein the dosage form is administered daily for at least 6 consecutive months.
48. The method of para 1, wherein the dosage form is an oral unit dose.
49. The method of para 48, wherein the oral dose unit is gelatin capsule, or caplet.
50. The method of para 1, wherein the subject has a logical memory baseline score greater than one standard deviation below a mean of a younger age group.
51. The method of para 1, wherein the subject is fifty years old or older.
52. The method of para 1, wherein the cognitive function is measured by determining CANTAB cognitive battery tests including working memory, memory retention, attention, and executive function measures.
53. The method of para 1, wherein the cognitive function improved is measured by the number of errors made on the PAL 6 pattern stage test.
54. The method of para 1, wherein the cognitive function improved is measured by the delayed logical memory test of the WMSIII, the CANTAB Verbal Recognition Memory test, the CANTAB Pattern Recognition test, the CANTAB Spatial Working memory test, Stockings of Cambridge test, or combinations thereof.
55. A method of reducing heart rate, comprising administering docosahexaenoic acid to a subject in need thereof about 0.8 g to about 4 g of docosahexaenoic acid (DHA) per day in a dosage form substantially free of eicosapentaenoic acid (EPA) and substantially free of arachidonic acid (ARA).
56. The method of para 55, wherein about 0.84 g to about 4 g of DHA is administered to the subject per day.
57. The method of para 55, wherein about 0.84 g to about 1.5 g of DHA is administered to the subject per day.
58. The method of para 55, wherein about 0.84 mg to about 1.0 g of DHA is administered to the subject per day.
59. The method of any one of paras 55 to 58, wherein the dosage form is administered once per day, twice per day, three times per day, four times per day, five times per day, six times per day, seven times per day, eight times per day, nine times per day, ten times per day, eleven times per day, or twelve times per day.
60. The method of paras 55 to 59, wherein the DHA is administered in at least one unit dose.
61. The method of para 60, wherein the unit dose comprises about 0.2 g to about 1 g ofDHA.
62. The method of para 61, wherein the unit dose has a total weight of about 0.20, 0.35, 0.40, 0.45, 0.50, 0.55, 0.60, 0.65, 0.70, 0.75, 0.80, 0.85, 0.90, 0.95, 1.0 g or 1.05 g.
63. The method of any one of paras 55 to 62, wherein the DHA comprises a DHA ester.
64. The method of para 63, wherein the DHA ester is an alkyl ester.
65. The method of para 64, wherein the DHA alkyl ester is a DHA methyl ester, ethyl ester, propyl ester, or combinations thereof.
66. The method of para 65, wherein the unit dose comprises about 430 mg to about 480 mg of DHA ethyl ester.
67. The method of para 65, wherein the unit dose comprises about 860 mg to about 950 mg of the DHA ethyl ester.
68. The method of para 67, wherein the unit dose comprises about 870 mg to about 930 mg of the DHA ethyl ester.
69. The method of para 55, wherein the heart rate is reduced about 1 beat per minute to about 10 beats per minute.
70. The method of para 55, wherein the heart rate is reduced about 2 beats per minute to about 4 beats per minute.
71. The method of para 55, further comprising selecting a subject in need of reduction in the heart rate.

## Claims

1. Docosahexaenoic acid (DHA) for use in reducing the heart rate in a subject,
wherein DHA is administered at a dosage of about 0.8 g to about 4 g per day in a dosage form substantially free of eicosapentaenoic acid (EPA) and substantially free of arachidonic acid (ARA).

2. DHA for use according to claim 1, wherein about 0.84 g to about 4 g of DHA is administered to the subject per day.

3. DHA for use according to claim 2, wherein the heart rate is reduced about 1 beat per minute to about 10 beats per minute.

4. DHA for use according to claim 3, wherein the heart rate is reduced about 2 beats per minute to about 4 beats per minute.

5. DHA for use according to claim 1, further comprising selecting a subject in need of reduction in the heart rate.

6. DHA for use according to claim 1, wherein the dosage form comprises a unit dose of about 860 mg to about 950 mg of DHA and wherein the dosage form comprises a DHA ethyl ester.

7. DHA for use according to claim 1, wherein the dosage form is an oral unit dose.
